# EUROPEAN PATENT SPECIFICATION

(11) **EP 0 326 120 B1**
(45) Date of publication and mention of the grant of the patent: **15.04.1998**
(21) Application number: 89101295.7
(22) Date of filing: 25.01.1989
(51) Int. Cl.: A61K 45/00, C12P 21/00, C12N 15/00

(54) **Human IFN-beta2/IL-6, its purification and uses**
Menschliches IFN-beta2/IL-6, dessen Reinigung und Verwendungen
IFN-bêta2/IL-6 humain, sa purification et ses utilisations

(30) Priority: 26.01.1988 IL 85204; 13.05.1988 US 193580; 14.11.1988 IL 88375; 14.11.1988 IL 88376
(43) Date of publication of application: 02.08.1989
(73) Proprietor: YEDA RESEARCH AND DEVELOPMENT COMPANY LIMITED, Rehovot 76100 (IL)
(72) Inventor: Revel, Michel, Rehovot (IL); Rubinstein, Menachem, Givat Shmuel (IL); Mory, Yves, Rehovot (IL); Chen, Louise, Ramat Aviv (IL); Novick, Daniela, Rehovot (IL); Michalevicz, Rita, Petah Tikva (IL)
(74) Representative: Grünecker, Kinkeldey, Stockmair & Schwanhäusser Anwaltssozietät

(56) References cited:
- EP-A- 0 119 859
- FEBS LETTERS, vol. 239, no. 2, November 1988, Elsevier Science Publishers BV (Biomedical Division); L. CHEN et al., pp. 299-304
- CHEMICAL ABSTRACTS, vol. 102, no. 15, 15 April 1985, Columbus, OH (US); D. NOVICK et al., p. 454, AN 129976b
- BIOLOGICAL ABSTRACTS, vol. 85, no. 5, 1988, Biological Abstracts Inc.; M. SUGI et al., AN 47888

## Description

### FIELD OF THE INVENTION

The present invention relates to monoclonal antibodies to human interferon-β2 (IFN-β2), to hybridoma cell lines producing said monoclonal antibodies and to a method for purification of interferon-β2 employing said monoclonal antibodies.

### BACKGROUND OF THE INVENTION

Human interferon-β2 was first described and cloned from double-stranded RNA-induced human fibroblasts as a β-type interferon-like activity in U.K. Patent 2063882 of filing date 19.11.80 and the recombinant protein expressed by CHO cells was described in European Patent Application 220,574 of 10.10.86, both of the same applicant.

This cytokine has multiple functions and activities and it has been shown to be identical to several proteins described later on in the literature and identified by other biological activities, namely B-cell differentiation or stimulatory factor (BCDF or BSF-2), also named interleukin-6 (IL-6) (T. Hirano et al. (1986) Nature 324, pp. 73-76), hybridoma/plasmacytoma growth factor (HGF or HPGF) (J. Van Damme et al. (1987) J.Exp.Med. 165, pp. 914-919), hepatocyte stimulatory factor (HSF) (J. Gauldie et al. (1987) Proc.Natl.Acad.Sci.U.S.A. 84, pp. 7251-7255), 26-kDa protein inducible in human fibroblast cells (Haegeman et al. (1986) Eur.J.Biochem. 159, pp. 625-632), and a monocyte-derived human B-cell growth factor that stimulates growth of Epstein-Barr Virus (EBV)-transformed human B-cells (G. Tosato et al. (1988) Science 239, pp. 502-504). The protein will be designated hereinafter in this application as IFN-β2/IL-6 or IFN-β2.

Natural and recombinant IFN-β2 produced by mammalian, e.g. CHO cells appear in several forms modified by various glycosylation and phosphorylation processes (A. Zilberstein et al. (1986) EMBO J. 5, pp. 2529-2537; L.T. May et al. (1988) J.Biol.Chem. 263, pp. 7760-7768; L.T. May et al. (1988) Biophys.Biochem.Res.Commun. 152, pp. 1144-1148). Thus immunoblots of denaturating gels may show bands of about 23, 26, 45 and 66 Kd forms. The removal of the carbohydrate moiety of the glycosylated protein yields a smaller form of about 20 Kd which still retains most or all of its biological activity.

### SUMMARY OF THE INVENTION

According to the present invention, there is provided a monoclonal antibody capable of specifically binding to natural human interferon-β2 (IFN-β2) as well as to recombinant human IFN-β2 expressed by mammalian cells and recombinant human IFN-β2 expressed by bacterial cells, capable of inhibiting the biological activity of human IFN-β2 when bound thereto, and capable of releasing IFN-β2 bound thereto upon elution with 50 mM citric acid.

In a preferred embodiment, the monoclonal antibody according to the present invention is of class IgG1. It is preferred that the monoclonal antibody is produced by a hybridoma cell obtained by fusion of myeloma cells with spleen cells from a mouse previously immunized with recombinant human IFN-β2 expressed by bacterial cells or with a recombinant fusion protein including human IFN-β2 expressed by bacterial cells, said hybridoma cell being selected for the production of a monoclonal antibody which binds to recombinant human IFN-β2 expressed by mammalian cells. Preferably, myeloma cells are murine myeloma cells and the spleen cells are from a mouse immunized with a recombinant fusion protein including human IFN-β2. With respect to the fusion protein, a preferred example is a fusion protein comprising protein A-IFN-β2. In a most preferred embodiment the monoclonal antibody is expressible by hybridoma cell line CNCM I-813 (HB2 34-1).

The present invention also provides a hybridoma cell capable of expressing the monoclonal antibody. Preferably, said hybridoma cell is obtained by fusion of myeloma cells with spleen cells from a mouse previously immunized with recombinant human IFN-β2 expressed by bacterial cells or with a recombinant fusion protein including human IFN-β2 expressed by bacterial cells, said hybridoma cell being selected for the production of a monoclonal antibody which binds to recombinant human IFN-β2 expressed by mammalian cells. In a preferred embodiment, the myeloma cells are murine myeloma cells and the spleen cells are derived from a mouse previously immunized with a recombinant fusion protein including human IFN β2. In a preferred embodiment, the fusion protein is protein A-IFN-β2. An example of such a hybridoma cell is CNCM I-813 (No.34-1).

The monoclonal antibody according to the present invention can be produced by a method which comprises:-
(a) immunizing mice with human IFN-β2 or with a fusion protein comprising human IFN-β2, wherein the source of said IFN-β2 or fusion protein is one of the sources selected from the group consisting of natural protein, recombinant protein produced in bacterial cells, and recombinant protein produced in mammalian cells;
(b) fusing the spleen cells from said mice with murine myeloma cells in the presence of a suitable fusion promoter;
(c) testing the supernatant of the cultured fused cells for the presence of the desired monoclonal antibodies with human IFN-β2 from a different one of said sources than that of the IFN-β2 used in step (a) above; and
(d) selecting and cloning the hybridoma cell line producing the desired monoclonal antibodies, and either (i) culturing the selected hybridoma cell line in a suitable growth medium and recovering the desired monoclonal antibodies from the supernatant, or (ii) injecting the selected hybridoma cell line into mice and recovering the desired monoclonal antibodies from the ascitic fluid of said mice.

In a preferred embodiment, in step (a) the mice are immunized with recombinant human IFN-β2 expressed by bacterial cells or with a recombinant fusion protein including human IFN-β2 expressed by bacterial cells and the hybridoma supernatants of step (c) are tested with recombinant human IFN-β2 expressed by mammalian cells. In the most preferred embodiment, in step (a) the mice are immunized with Protein A-IFN-β2 expressed by *E.coli* and the hybridoma supernatants of step (c) are tested with IFN-β2 expressed by CHO cells.

Also within the scope of the present invention is a method for producing anti-IFN-β2 monoclonal antibodies which comprise
(a) culturing hybridoma CNCM I-813 in a suitable growth medium and recovering the monoclonal antibodies from the supernatant of said hybridoma,
   or
(b) injecting hybridoma CNCM I-813 in a mouse and recovering the monoclonal antibodies from the ascitic fluid of said mouse.

Furthermore, the present invention comprises a method for immunopurification of human biologically active IFN-β2 comprising passing a sample containing human IFN-β2 through an immunoadsorbent column comprising a monoclonal antibody in accordance with claim 1 bound to a solid phase support, washing the column, and eluting IFN-β2 from the column.

### BRIEF DESCRIPTION OF THE DRAWINGS

- Figure 1 shows the nucleotide sequence and the amino acid sequence of IFN-β2.
- Figure 2 shows the construction of plasmid pSVβ₂HB.
- Figure 3 shows the construction of plasmids pRIβ₂802 and pRIβ₂604.
- Figure 4 illustrates the analysis of IFN-β2 preparations by Western blotting.
- Figure 5 shows silver stain analysis of SDS-PAGE of IFN-β2 eluted fractions.
- Figure 6 shows the construction of plasmid pSVβ₂29.
- Figure 7 shows the HGF activity of unbound fractions containing IFN-β2 produced by CHO clone A2-5-10 after affinity chromatography with monoclonal antibody 34-1.
- Figure 8 shows the HGF activity of elution fractions containing IFN-β2 produced by CHO clone A2-5-10 after affinity chromatography with monoclonal antibody 34-1.
- Figure 9 illustrates the construction of plasmids pTLβ₂501 and pKKβ₂7.
- Figure 10 shows silver stain analysis of SDS-PAGE of E. coli IFN-β2 after immunoaffinity purification and chromatography with S-Sepharose.
- Figure 11 shows inhibition of breast carcinoma cell line T47D colony formation by E. coli IFN-β2.
- Figure 12 shows results of breast carcinoma T47D and MCF-7 cells clonogenic assay with E. coli IFN-β2.
- Figure 13 shows differentiation of myeloleukemic M1 cells induced by E. coli IFN-β2.
- Figure 14 shows growth and (2'-5') Oligo A synthetase induction in myeloleukemic M1 cells treated by IFN-β2 (in HGF units/ml).
- Figure 15 shows effect of IFN-β2 on growth of hematopoietic colonies from normal human bone marrow.

### DETAILED DESCRIPTION OF THE INVENTION

The anti-IFN-β2 monoclonal antibodies of the invention are produced from a hybridoma cell line obtained by fusion of murine myeloma cells with spleen cells from a mouse previously immunized with IFN-β2 or with a fusion protein comprising IFN-β2, e.g. Protein A-IFN-β2 fusion protein.

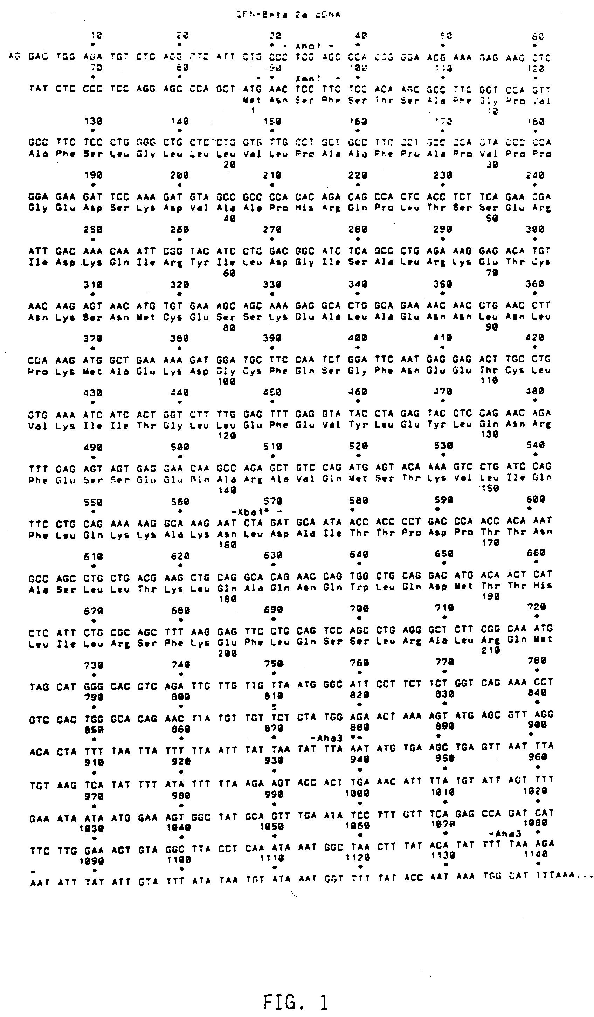

A plasmid for the expression in E. coli of a Protein A-IFN-β2 fusion protein can be constructed and used for obtaining monoclonal antibodies. The complete translated sequence of the cDNA coding for human IFN-β2 of Fig. 1 was fused, in phase, to the 3′ end of the coding sequence for the staphylococcal Protein A affinity tail (Uhlen et al. (1984) J.Biol.Chem. 259, p. 1695). For efficient expression in E. coli the hybrid gene was fused to the strong lambda P_{R} promoter.

The resulting Protein A-IFN-β2 fusion protein was purified and used to immunize mice. After six injections of the purified protein into mice, positive sera were tested for their binding titer in a solid phase radio-immunoassay (SRIA) and for the specificity of binding by Western blots. Spleen cells derived from a mouse showing the highest binding titer (dilution 1:25,000) were fused to mouse myeloma cells. The fusion of the cells is done in the presence of a suitable fusion promoter of those known in the art. The fused cells are then cultured in separate wells. The supernatant of each well is then tested for the presence of the desired monoclonal antibody capable of specifically binding to IFN-β2, preferably with IFN-β2 from a different source than the IFN-β2 used for the immunization of the mice. Thus, if a Protein A-IFN-β2 fusion protein expressed by E. coli cells is used to immunize the mice, then the screening of the monoclonal antibodies is performed with IFN-β2 produced by CHO cells. This prevents cross-reaction of the Protein A and of any E. coli contaminants in the antigen preparations used for injection with some of the monoclonal antibodies during the screening. For the SRIA, crude supernatants of CHO cells, harboring a plasmid containing the human IFN-β2 gene under the control of the SV40 early promoter and expressing high levels of this gene but no Protein A or any bacterial antigen, were bound to a solid support and reacted with supernatants of the hybridomas and with [¹²⁵I] goat antimouse antibodies.

The hybridomas were screened by the SRIA and several positive clones were isolated and characterized. The positive clones producing the desired antibodies are then selected and subcloned and either cultured in a suitable growth medium or injected into mice, and the desired monoclonal antibodies are then recovered from the supernatant of the cultured cells or from the ascitic fluid of said mice, respectively.

The monoclonal antibody obtained is bound to a solid phase support contained within a column. Any suitable gel matrix known in the art may be used to immobilize the monoclonal antibody e.g. agarose-polyacrylhydrazide. Crude preparations of human IFN-β2 preparations are loaded on the column and IFN-β2 is eluted from the gel by a change in pH or ionic strength. In a preferred embodiment, the fractions containing IFN-β2 are eluted with a buffer pH 2 and neutralized immediately after elution with a buffer pH 8.5.

The crude natural IFN-β2 preparations purified according to the invention are obtained from induced fibroblast cells together with IFN-β1.

The recombinant IFN-β2 produced by CHO cells to be purified is obtained according to the process described in A. Zilberstein et al. (1986) EMBO J. 5, pp. 2529-2537.

An unglycosylated polypeptide comprising the amino acid sequence of IFN-β2 can be obtained by recombinant DNA techniques. Human DNA, particularly cDNA coding for a polypeptide comprising the amino acid sequence for IFN-β2, is fused through the coding region to a strong bacterial promoter, such as hybrid tryp-lac promoter, and this fused DNA molecule is inserted into a suitable plasmid so as to obtain a recombinant vector comprising said DNA sequence and regulatory regions which are positioned in such a way that expression of said polypeptide in bacterial cells is possible. Bacterial cells, e.g. E. coli, are transformed by said recombinant plasmids and cultured in order to express the desired polypeptide, which is subsequently recovered and purified.

The IFN-β2 molecule as described is a polypeptide comprising 212 amino acids. At the N-terminus of the protein may be found the sequences Ala²⁸-Pro-Val-Pro-Pro- or -Pro²⁹-Val-Pro-Pro- or -Val³⁰-Pro-Pro- of Fig. 1. IFN-β2 cDNA can be fused at the Pro²⁹ codon to a Met initiator condon and a tryp-lac promoter. Other sequences can be used as long as the polypeptide has IFN-β2/IL-6 activity.

DNA vectors can be constructed by standard procedures. Plasmid DNAs were purified by banding in CsCl-ethidium bromide gradients. DNA restriction fragments separated by electrophoresis in agarose or polyacrylamide gels were purified on DE-52 columns. Restriction endonucleases (Boehringer, New England Biolabs), T4 DNA ligase (New England Biolabs), the large fragment of E. coli DNA polymerase (Boehringer) and T4 polynucleotide kinase (Pharmacia), were used as recommended by the suppliers. E. coli transformation was carried out with frozen competent bacteria (D.A. Morrison (1979) Methods Enzymol. 79, pp. 326-331) using strains HB101 ATCC 33694, JM101 ATCC 33876, N4830-1 (Gootesman et al. (1980) J.Mol.Biol. 140, p. 57) and JM105 (Messing et al. (1981) Nucleic Acids Res. 9, pp. 309-321).

The hematopoietic effect of IFN-β2 is disclosed herein, as well as its use in the inhibition of breast carcinoma cell growth, in the growth inhibition and differentiation of myeloleukemic cells and in the induction of Complement Factor B in fibroblasts. Thus human IFN-β2 can be used as active ingredient of pharmaceutical compositions for the treatment of breast cancer, leukemia, infectious diseases and bone marrow progenitor cell disorders.

The invention will now be illustrated by examples, without delimiting its scope. Where examples do not relate to subject matter claimed, these are provided for information purposes.

### Example 1. Preparation of Protein A-IFN-β2 fusion protein

### A) Construction of plasmids pRIβ₂802 and pRIβ₂604

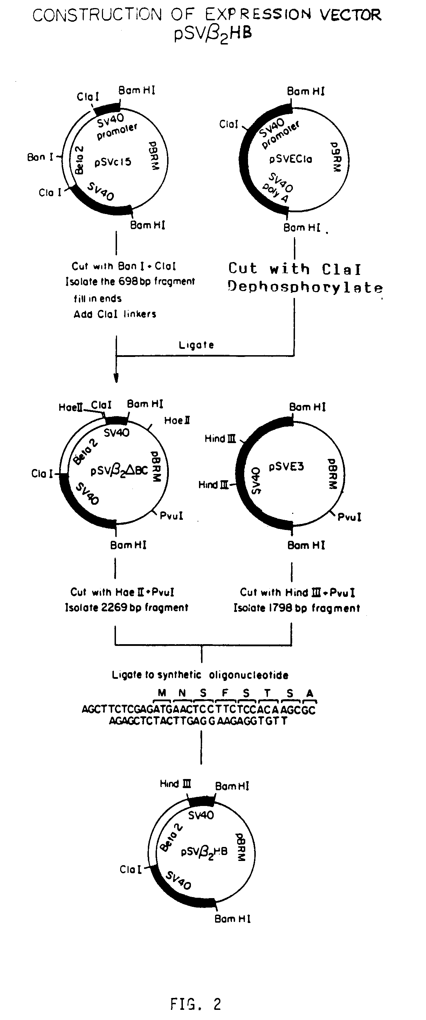

Plasmid pSVβ₂HB (Fig. 2) is one of the vectors used for constitutive expression of the IFN-β2 gene in CHO cells under the strong SV40 early promoter and is derived from plasmid pSVCIFβ₂ (A. Zilberstein et al. (1986) EMBO J. 5, pp. 2529-2537) by removing all the 5' and 3' non-coding sequences of the cDNA by standard cloning techniques.

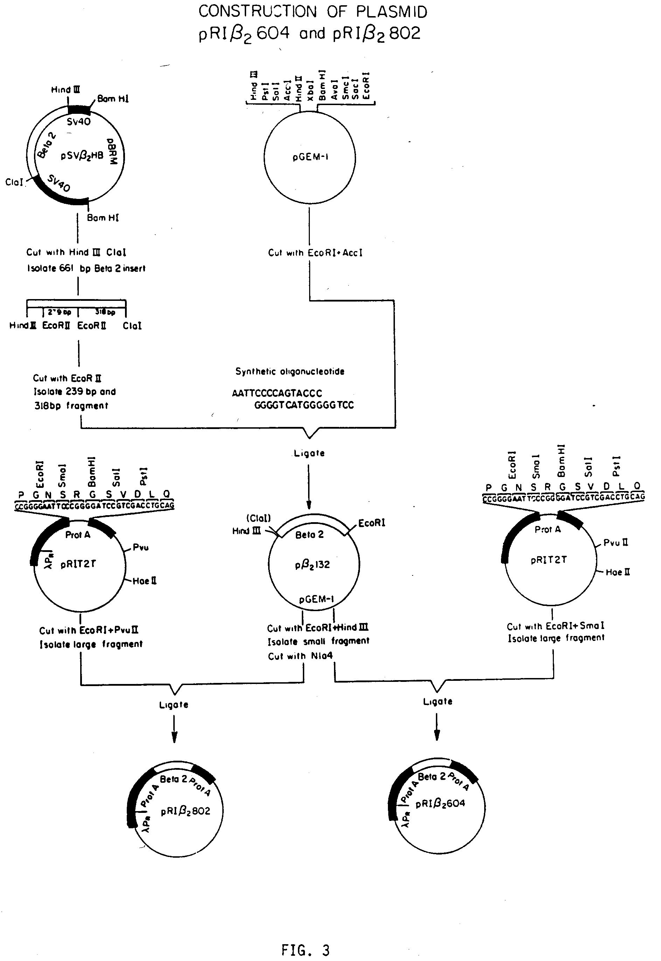

The 661 bp cDNA insert coding for IFN-β2 was excised from plasmid pSVβ₂HB as a 661 bp Hind III/Cla I fragment and digested with Eco RII. The resulting five fragments were separated on a preparative agarose gel. The three small fragments of 55, 12 and 37 bp coding for the signal peptide sequence and for the first three amino acids of the mature protein were discarded and the two fragments of 239 and 318 bp were recovered from the gel. In order to restore the sequence coding for the first amino acids and to maintain the Protein A frame, a double stranded synthetic oligonucleotide was prepared (sequence shown in Figure 3) and ligated together with the 239 bp and 318 bp fragments into plasmid pGEM-1 previously digested with Eco RI and Acc I. The resulting plasmid was called pβ₂132 (Fig. 3) and contains the whole IFN-β2 sequence preceded by an asparagine and a serine codon within the multiple cloning site of plasmid pGEM-1. The asparagine and serine codons are the two codons at the unique Eco RI site (at the 3′ end of the Protein A gene) of plasmid pRIT2T (Pharmacia) that was used for subsequent cloning. Plasmid pβ₂132 was digested with Eco RI and Hind III and the complete IFN-β2 cDNA sequence was isolated and introduced into plasmid pRIT2T digested either with Eco RI and Pvu II or with Eco RI and Sma I restriction endonucleases for the obtention of the plasmids pRIβ₂802 and pRIβ₂604, respectively (Fig. 3).

### B) Production of Protein A-IFN-β2 fusion protein and its purification

Strain E. coli N4830-1 (Gootesman et al. (1980) J.Mol.Biol. 140, p. 57) was transformed with recombinant plasmids pRIβ₂802 and pRIβ₂604 giving origin to new microorganisms E. coli N4830-1/pRβ₂802 and E. coli N4830-1/pRβ₂604, respectively.

Diluted cell cultures of the microorganisms were grown overnight at 30°C in M9 medium containing ampicillin to early stationary phase, then incubated at 42°C for 90 minutes, cooled and harvested by centrifugation. After repeated resuspension and centrifugation, 20% SDS was added to a final concentration of 1% and 10M urea to a final concentration of 8M, and the extract containing the expressed Protein A-IFN-β2 fusion protein was dialyzed against TST (50 mM Tris pH 7.6, 150 mM NaCl and 0.05% Tween 20). The clear supernatant after dialysis was applied to the IgG Sepharose 6 Fast Flow (FF) equilibrated column. After loading on the column the gel was washed and the bound fusion protein was eluted with 0.5 M NH₄COOH, pH 3.4 and lyophilized directly without prior dialysis.

### Example 2. Preparation of anti-IFN-β2 monoclonal antibodies

### A) Immunization of mice and cell fusion

Three-month old female Balb/c mice were first injected with the partially purified Protein A - IFN-β2 fusion protein obtained in Example 1 above (10µg/mouse, emulsified in complete Freund's adjuvant). Three weeks later the mice were given a subcutaneous boost with the fusion protein in solution. Four additional injections were given at 10 days intervals. The mouse showing the highest binding titer (Table 1) and the strongest signal in Western blot analysis received an intra- peritoneal injection of the fusion protein and three days later its splenic lymphocytes (150 x 10⁶ cells) were fused with 30 x 10⁶ NSO/l myeloma cell line. The fused cells were distributed into microculture plates (3 x 10⁴ cells/well) and selected for hybridoma growth. Hybridomas that were found to secrete anti-IFN-β2 antibodies were clones and recloned by the limiting dilution technique.

### B) Screening for anti-IFN-β2 monoclonal antibody-producing specific hybridomas

Hybridoma supernatants were tested for the presence of anti-IFN-β2 antibodies by a solid phase radioimmunoassay (SRIA). PVC microtiter plates (Dynatech Laboratories, Alexandria, VA) were coated with a crude, serum-free supernatant of CHO cells secreting IFN-β2 (80µg/well). Following an incubation of 2 hrs at 37°C or 16 hrs at 4°C the plates were washed twice with PBS containing BSA (0.5%) and Tween 20 (0.05%) and blocked in washing solution for 2 hrs at 37°C. Hybridoma culture supernatants (50 µg/well) were added and the plates were incubated for 4 hrs at 37°C. The plates were then washed three times with the washing solution and ¹²⁵I-goat anti-mouse (Fab′)₂ (50µl, 10⁵ cpm) was added for further incubation of 16 hrs at 4°C. The plates were washed 4 times and individual wells were cut and counted in a gamma counter. Samples giving counts that were at least four times higher than the negative control value were considered positive (Table 1).

**Table 1**

| Screening of hybridomas by SRIA | | | |
|---|---|---|---|
| Sample | | dilution | cpm |
| Immune serum (mouse) | | 1:4000 | 2800 |
| negative control (mouse) | | 1:4000 | 100 |
| Hybridoma | 12 | | 4000 |
| | 12 | 1:125 | 300 |
| | 27 | | 1100 |
| | 28 | | 2200 |
| | 34 | | 6200 |
| | 34-1 | 1:2500 | 1000 |
| | 38 | | 2600 |
| | 48 | | 1500 |
| | 102 | | 1200 |
| | 117 | | 1400 |
| | 123 | | 1100 |
| | 125 | | 1600 |
| | 132 | | 5400 |
| | 136 | | 2700 |
| | 154 | | 1500 |
| | 157 | | 2400 |
| negative hybridoma | | | 200 |
| Ascitic fluid | | 1:62,000 | 1400 |
| Ascitic fluid negative | | 1:12,000 | 300 |

As can be seen from Table 1, fourteen anti-IFN-β2 hybridomas were selected using the SRIA. Hybridoma No. 34-1, sub-cloned from hybridoma 34, was further characterized and was found to belong to IgG1 class. Hybridoma 34-1 was deposited with the Collection Nationale des Cultures de Microorganismes - CNCM, Institute Pasteur, Paris, on 14.11.88. It was accorded No. I-813.

Hybridoma 34-1 was found suitable for Western blotting and for affinity purification of natural and recombinant IFN-β2 expressed both by E. Coli and by CHO cells. It was used in the following experiments.

### Example 3. Applications of the anti-IFN-β2 monoclonal antibodies

### A) Western blotting

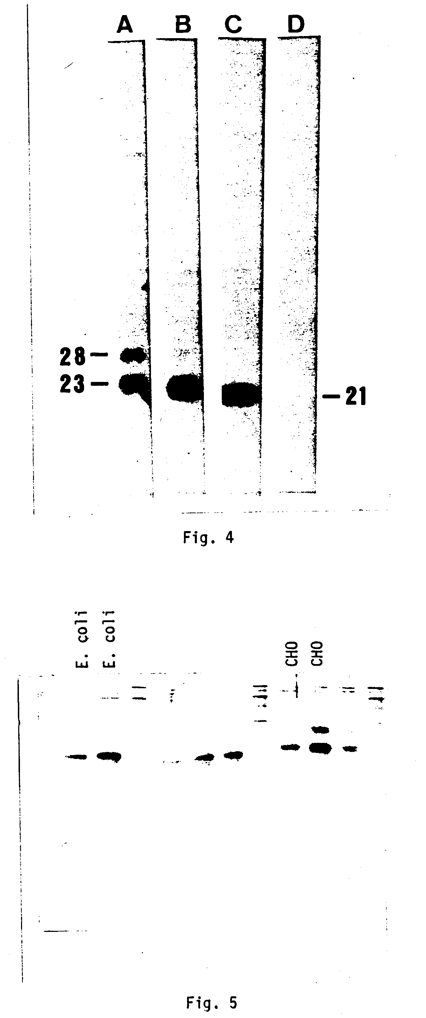

Samples of crude preparations of either natural or recombinant IFN-β2 expressed by CHO and E. coli cells were analyzed by SDS-PAGE under reducing conditions and electroblotted onto nitrocellulose sheets (BA85, Schleicher and Shuell). Following electroblotting the sheet was incubated overnight with a blocking buffer (5% non-fat milk in PBS containing 0.05% Tween 20 and 0.02% sodium azide) and then for 2 hrs at room temperature with the anti-IFN-β2 antibody No. 34-1. Following washing in 0.05% Tween 20 in PBS, the nitrocellulose was incubated for 3 hrs at room temperature with ¹²⁵I-goat anti-mouse serum (0.7 x 10⁶ cpm/ml in the blocking buffer). The sheet was then washed, dried and autoradiographed. The results are shown in Fig. 4: Lane A: natural IFN-β2; Lane B: recombinant IFN-β2 of CHO cells; Lane C: recombinant IFN-β2 of E. coli cells; Lane D: recombinant IFN-β1 of CHO cells (comparison).

### B) Affinity chromatography of IFN-β2 preparations

Ascitic fluids of mice containing monoclonal antibodies secreted by hybridoma 34-1 were precipitated with ammonium sulfate (50% saturation) 16 hrs at 4°C. The precipitate was collected by centrifugation, redisolved in water and dialysed against saline. About 10 mg of immunoglobulins were bound to 1 ml agarose-polyacryl-hydrazide according to Wilcheck and Miron ((1974) Methods Enzym. 34, p.72). Crude preparations of either natural (fibroblast) or recombinant (E. coli or CHO) IFN-β2 (containing 0.5 M NaCl) were loaded at 4°C at a flow rate of 0.25 ml/min. The column was washed with 30 column volumes of 0.5 M NaCl in PBS. IFN-β2 was eluted by 50 mM citric acid buffer, pH 2 (8 x 1 column volume fractions) and immediately neutralized by 0.1 M Hepes buffer, pH 8.5.

Crude recombinant IFN-β2 (E. coli extract depleted from DNA) was loaded on 1 ml of the anti-IFN-β2 column. Purification of 1000 fold was achieved in one step, and the recovery of IFN-β2 was 100% (Table 2). The procedure was scaled by using 8 ml affinity column. The capacity of the column was 400 µg pure IFN-β2 per 1 ml of column. Silver stain analysis of SDS-PAGE of the eluted fractions revealed a major band of a M.W. of 21,000 and some minor contaminants of a higher M.W. (Fig. 5). When crude recombinant IFN-β2 (CHO) was loaded on 1 ml affinity column, purification was achieved in one step with a recovery of 100%. Silver stain analysis of SDS-PAGE of the eluted fractions revealed two major bands of 23 kDa and 28 kDa, both belonging to the glycosylated forms of IFN-β2 (Fig. 5). The same bands were obtained when natural IFN-β2 (foreskin fibroblasts) was immunoaffinity purified.

**Table 2**

| Source of IFN-β2 | Sample | HGF units/ml x 10⁻⁴ | Prot.conc. mg/ml | Spec.act. units/mg | Purif. fold | Recovery % |
|---|---|---|---|---|---|---|
| E. coli | load | 1.4 | 4.1 | 3400 | | |
| | efluent | 0.54 | 4.1 | 1300 | | |
| | eluate | 25 | 0.09 | 3.6 x 10⁶ | 1060 | 100 |
| CHO | load | 0.2 | | | | |
| | efluent | 0.06 | | | | |
| | eluate | 2.7 | 0.14 | 0.2 x 10⁶ | | 100 |
| Foreskin fibroblast | load | 0.014 | 1.46 | 95 | | |
| | efluent | 0.014 | - | | | |
| | eluate | 0.014 | 0.008 | 17 x 10⁴ | 180 | 100 |

### Example 4. Monitoring of IFN-β2 produced by CHO cells

IFN-β2 produced and secreted in one liter of culture medium by CHO clones was quantitated using monoclonal antibody 34-1 for purification by immunoaffinity.

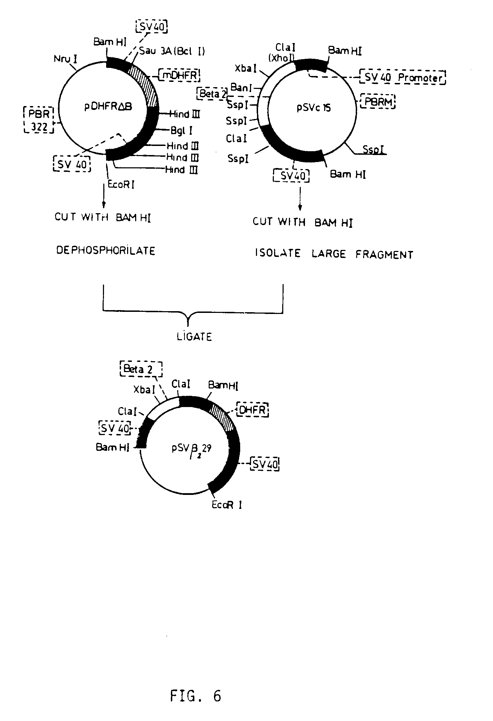

Clone A2-5-10 is a CHO clone obtained by transfection of CHO cells with plasmid pSVβ₂29 (Fig. 6) and selection with 50 nM methotrexate (MTX).

Plasmid pSVβ₂29 was obtained as follows: a DNA fragment containing the sequence coding for IFN-β2 fused to the early promoter of SV40 and to the SV40 polyadenylation site was excised from plasmid pSVc15 as a 2.5 kb BamHI fragment. Plasmid pSVc15, one of the vectors previously used for constitutive expression of IFN-β2 in CHO cells under the strong SV40 early promoter, was derived from plasmid pSVCIFβ₂ (A. Zilberstein et al. (1986) EMBO J. 5, pp. 2529-2537) by removing all the sequence 5′ to the XhoI site of the IFN-β2 cDNA. This BamHI fragment was cloned into the BamHI site of a pDHFR plasmid containing a mouse DHFR cDNA fused to the SV40 early promoter and a splicing region of mouse IgG gamma-2a.

The clone A2-5-10 was grown to confluency in roller bottles. The culture medium was changed to a low (2%) fetal calf serum and collected 24 hours after the change. One liter of culture was concentrated to 45 ml and loaded on the monoclonal antibody affinity column prepared in Example 3B. The column was extensively washed and the bound IFN-β2 was eluted with 50 mM citric acid pH 2, in four fractions of one ml each. The IFN-β2 purified in this way seems to be homogeneously pure as indicated by silver stain analysis of SDS-PAGE (Figure 5). The amount of IFN-β2 protein recovered from one liter of culture was 469 µg.

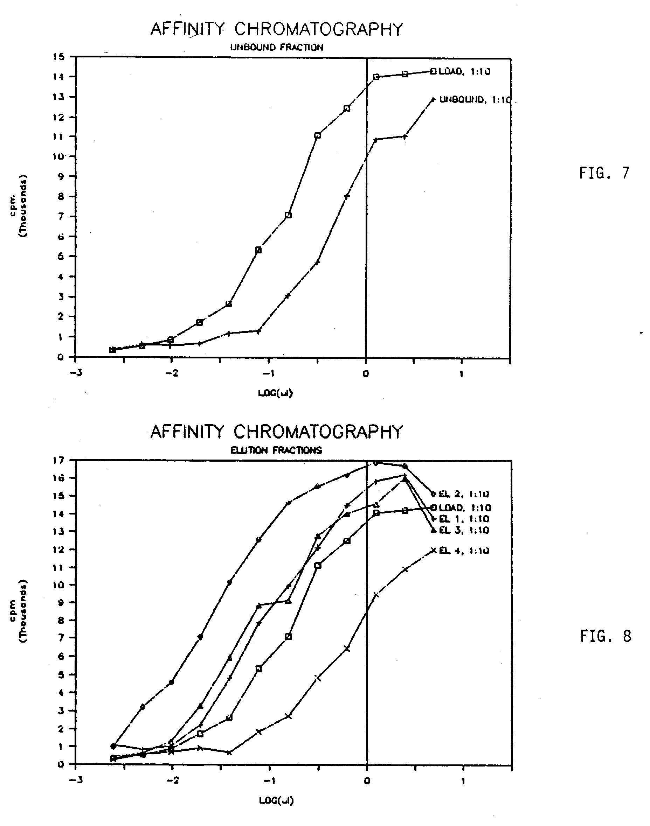

The amount of IFN-β2 in each fraction was estimated by measuring the hybridoma growth factor (HGF) activity of the protein in the crude preparation and in the different fractions of the affinity column. About 40% of the IFN-β2 loaded on the column was recovered in the unbound fraction (Figure 7), while the remaining activity was recovered in fractions eluted with pH 2 (Figure 8) with a peak in elution 2. These results indicate that, under the conditions described above, clone A2-5-10 produces about 800 µg/l of IFN-β2.

The specific acitivity of the IFN-β2 produced and secreted by the CHO clone A2-5-10 was determined by measuring the HGF activity and the protein concentration in each of the purified fractions of the immuno-affinity column. One unit of HGF is defined as the amount of protein that gives 50% of the maximal effect in the assay. The HGF activity was assayed in 0.1 ml cultures of murine plasmacytoma T1165 cells, treated for 24 hours and pulsed for 16 hours with [³H]thymidine as described by Nordan R.P. and Potter M. (1986) Science 233, pp. 566-568. Table 3 summarizes the results of such an analysis. The specific HGF activity of IFN-β2 in the three fractions, eluted from the affinity chromatography column, ranged from 1.18x10⁶ to 2.1x10⁶ with an average of 1.47x10⁶.

**Table 3**

| Specific Activity of IFN-β2 | | | |
|---|---|---|---|
| Fraction | HGF activity U/ml | Protein concentration mg/ml | Specific activity U/ml |
| Elution 1 | 128,000 | 0.108 | 1.18x10⁶ |
| Elution 2 | 333,000 | 0.239 | 1.39x10⁶ |
| Elution 3 | 166,000 | 0.079 | 2.10x10⁶ |
| Total | 627,000 | 0.426 | 1.47x10⁶ |

The elution fractions from the affinity chromatography column were pooled, dialyzed to 10mM acetate buffer pH 5 and loaded on a Mono S cation exchange column (Pharmacia). The column was washed with 10 mM acetate buffer pH 5 and then eluted using a linear sodium chloride gradient from 0 to 600 mM. HGF activity was determined in the different fractions. Activity coincided with the main peak of protein.

### Example 5. Preparation and purification of recombinant IFN-β2 produced by E. coli cells

### A) Construction of plasmids pTLβ₂501 and pKKβ₂7

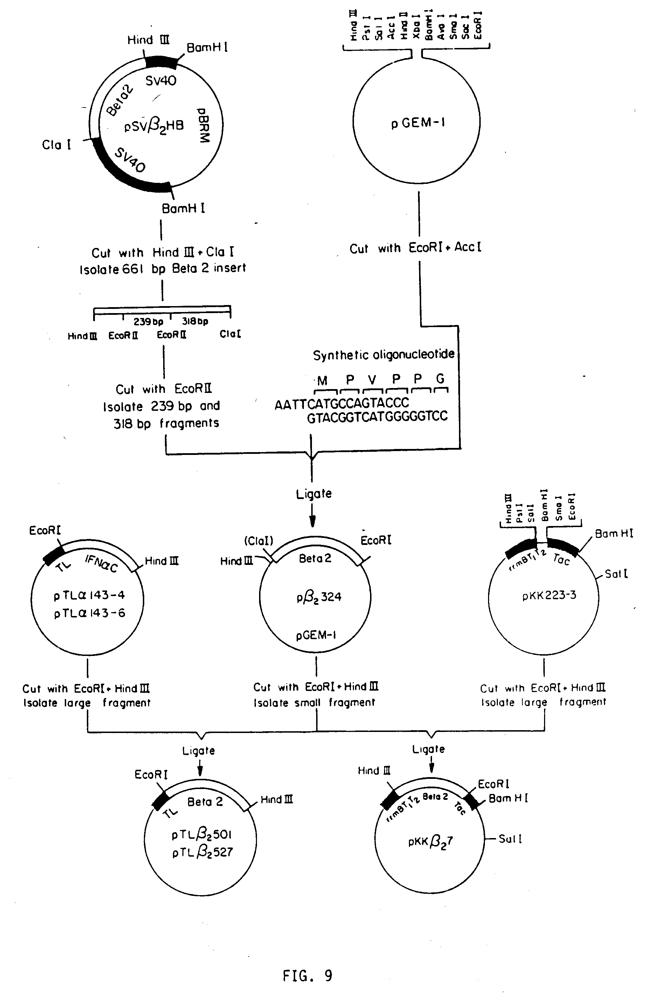

Plasmid pβ₂324 containing the whole IFN-β₂ sequence preceded by an ATG codon within the multiple cloning site of plasmid pGEM-1 ws prepared in the same way as plasmid pβ₂132 in Example 1A and Figure 3, except for the fact that a different synthetic oligonucleotide (Fig. 9) was used. Plasmid pβ₂324 was digested with Eco RI and Hind III and the complete IFN-β2 cDNA sequence was isolated and introduced into either one of plasmids pTLα143-4 (Y. Chernajovsky et al., (1983) Ann. N.Y. Acad. Sci. 413, pp. 88-96) or pKK223-3 (Pharmacia) digested with Eco RI and Hind III for the obtention of the plasmids pTLβ₂501 and pKKβ₂7 (Fig. 9).

### B) Expression of the biologically active IFN-β2 polypeptide in E. coli

Strain E. coli JM105 (described in J. Messing et al., (1981) Nucleic Acids Res. 9, pp. 309-321) was transformed with recombinant plasmid pKKβ₂7 giving origin to new microorganism E. coli JM105/pKKβ₂7 deposited with the ATCC under the Budapest Treaty on 17.12.1987 and assigned the number ATCC 67583. The transformation of strain E. coli JM101 (ATCC 33876) with the recombinant plasmid pTLβ₂501 gave origin to new microorganism E. coli JM101/pTLβ₂501 deposited with the ATCC under the Budapest Treaty on 17.12.1987 and assigned the number ATCC 67584. The microorganisms were culture, lysed and the extracts containing IFN-β2 were purified.

### C) Purification of E. coli IFN-β2 by chromatography

Nucleic acid-free bacterial extracts were obtained by polyethyleneimine precipitation and passage on DEAE cellulose. The effluent fractions were adsorbed onto S-Sepharose in 10 mM Na acetate buffer pH 5 (buffer A) and eluted with a 0-0.03 M NaCl gradient. Active fractions were pooled, dialyzed against buffer A, chromatographed on a Mono-S FPLC column and eluted with a 0-0.03 M NaCl gradient. HGF activity was followed during purification and coincided with the IFN-β2 protein revealed by immunoblots using polyclonal antibodies against a N-terminus peptide of IFN-β2. The final preparation showed a single band of about 20 kDa on SDS-polyacrylamide gel electrophoresis.

### D) Immunopurification of E. coli IFN-β2

For purification, 1-1.5 l of Dyno-mill extract obtained from 3-6 liter of fermentor culture were precipitated with polyethyleneimine, the solution was concentrated down to 100 ml (A4) or 500 ml (A8) and loaded on an 8 ml column of monoclonal antibody 34-1 (Ig from ascites, 8 mg Ig/ml column) in phosphate buffered saline (PBS) with 0.5M NaCl pH 7.0 (A4) or 1M NaCl (A8). After washing the column with the same buffer, elution was carried out by 50mM citric acid buffer pH 2 (A4) or with the same citric buffer and propylene glycol 25% (A8) and samples were immediately neutralized by addition of 0.1M Hepes buffer pH8.5. The results were as follows:

### A4: Immunopurification of E. coli IFN-β2:

| Fraction | Volume | Protein | HGF activity | HGF U/ml | HGF U/mg |
|---|---|---|---|---|---|
| Load eluted: | 90 ml | 5,700 mg | 29 million U | 325,000 | 5,100 |
| Total | 40 ml | 8.2 mg | 2.9 million U | | |
| (Tube 3) | 10 ml | 2.2 mg | | 110,000 | 500,000 |

### A8: Immunopurification of E. coli IFN-β2:

| Fraction | Volume | Protein | HGF activity | HGF U/ml | HGF U/mg |
|---|---|---|---|---|---|
| Load eluted: | 350 ml | 2,550 mg | 10 million U | 330,000 | 4,100 |
| Total | 37 ml | 5.2 mg | 22 million U | | 4.2 million |
| (Tube 3) | 9.5 ml | 2 mg | | 1.3 million | |

Following immunoaffinity, an S-Sepharose column was used as the final purification step. In a typical experiment, (S12), the input from pooled A4 fractions was dialyzed against 10mM acetate buffer pH 5, adsorbed and eluted by a gradient from 0.1-0.4 M NaCl. The peak eluted at 0.3 M. The results were as follows:

### S12: S-Sepharose after immunoaffinity

| Fraction | Volume | Protein | HGF activity | HGF U/ml | HGF U/mg |
|---|---|---|---|---|---|
| Input: | | | | | |
| A4 pool | 17.5 ml | 3.6 mg | 1.4 million U | | 0.5 million |
| pH5 dial. | 22.5 ml | 2.2 mg | ND | | |

| Eluted: | | | | | |
|---|---|---|---|---|---|
| Total peak | 4.5 ml | 0.6 mg | 0.9 million U | | 2 million |
| Tube 58 | 0.5 ml | 0.04mg | | 210,000 | 5.2 million |

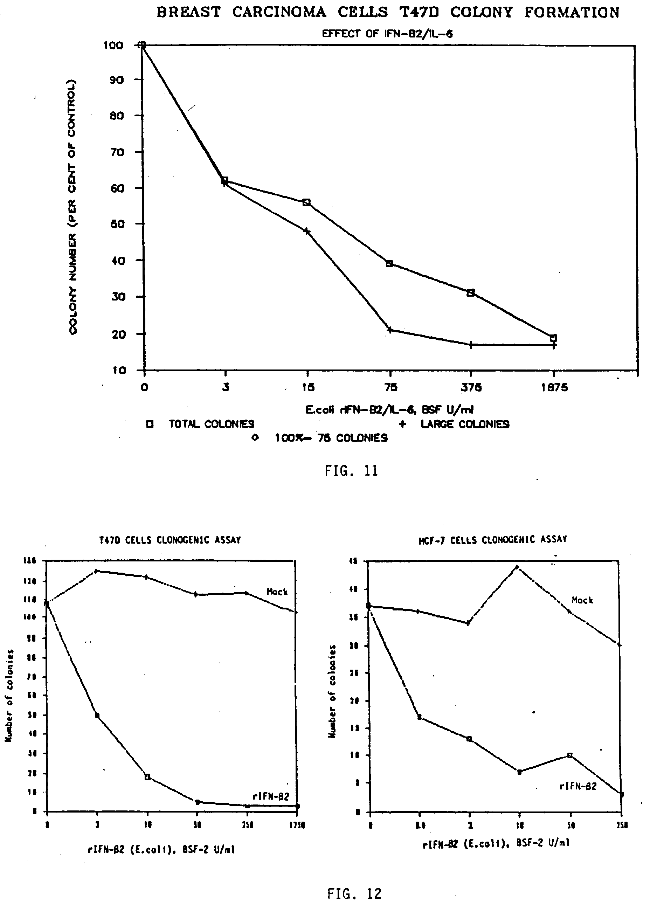

The yield in this step was 64%. The product was run on SDS polyacrylamide gel under reducing and non-reducing conditions and showed one single band at 21 Kd. (Fig. 10).

### Example 6. Growth inhibition of breast carcinoma cells

While IFN-β2/IL-6 stimulates growth of plasmacytoma/hybridoma cells (HGF activity), it is growth inhibitory on other cell types. We have studied colony formation by the T47D line of human Breast Ductal carcinoma cells (ATCC HTB 133), which is poorly inhibited by IFN-β1 but is sensitive to inhibition by IFN-β2. Fig. 11 shows inhibition of T47D colony formation in culture dishes by pure E. coli rIFN-β2/IL-6. The 50% inhibition is seen with 10-20 HGF U/ml. At doses over 100 U/ml, the remnant colonies are very small and appear to represent mainly growth arrested cells (insert). Inhibition of ³H-thymidine incorporation in semiconfluent T47D cells by E. coli and CHO rIFN-β2/IL-6 was found to be neutralized by the anti-IFN-β2 monoclonal antibody 34-1.

To investigate if the inhibition of ³H-thymidine incorporation in T47D cells represents a genuine antigrowth activity, we used a 15 day clonogenic assay (Fig. 12). A 50% decrease in the number of colonies of T47D cells was observed with 2 BSF-2 U/ml of E. coli IFN-β₂ and an almost complete inhibition of growth in these conditions was reached at 50 U/ml. The mock preparation had no inhibitory activity in this clonogenic assay (Fig. 12 a). In the same experiment we observed that IFN-β₁ at 500 antiviral U/ml produced no inhibition of T47D cell growth. Decreased colony formation was similarly observed in another breast carcinoma cell line MCF-7 (ATCC HTB 22) (Fig. 12 b). By ³H-thymidine incorporation, MCF-7 appears somewhat less sensitive than T47D cells.

For the clonogenic assay, T47D cells were seeded at 200 cells per well and MCF-7 at 1,000 cells per well in 6-well Costar plates in 1 ml RPMI 1640 with 10% fetal calf serum (FCS), insulin 0.2 U/ml, glutamine 2 mM, penicillin 100 U/ml, streptomycin 100 ug/ml. Cells received 24 hours later serial 5 fold dilutions of E. coli IFN-β2 or mock E. coli preparations at the same protein concentration. After 15 days the colonies were stained with crystal violet and counted under an inverted microscope. For DNA synthesis measurements, cells were seeded at 15-25 x 10³ per well of a 96-well microplate, and after 3 days FCS was removed for 24 hours and readded in fresh medium with serial 5 fold dilutions of E. coli IFN-β2 or mock preparations. After 16-24 hours, cells were labeled with 15 µCi/ml ³H-thymidine (25 Ci/mmol, Amersham) for 1 hour, washed twice with PBS, treated by 5% trichloroacetic acid (TCA) for 30 min at 4°C and washed 3 times with 5% TCA. The precipitate was dissolved in 0.1 ml of 0.2 M NaOH at 37°C for 30 minutes, neutralized by 0.01 ml of 2M HCl and counted in a Tricarb counter with toluene scintillator and Lumax (3:2 v/v). Similar results were obtained without serum starvation, ³H-thymidine incorporation was lower but inhibition was the same.

Extracts containing IFN-β2 were assayed for BSF-2 activity as measured by the stimulation of IgG secretion by CESS cells in response to treatment with said extracts (A. Muraguchi et al., (1981) J. Immunol. 128, pp. 1296-1301; T. Hirano et al., (1985) Proc. Natl. Acad. Sci. USA 82, pp. 5490-5494) and for HGF activity as measured by the ability of the extracts to support the growth of plasmacytoma cell line T1165 (R.P. Nordan and M. Potter, (1986) Science 233, pp. 566-569). Stimulation of ³H-thymidine incorporatio in T1165 cells and of IgG secretion by CESS cells showed half-maximum at a dilution of 1:12,500 which was therefore defined as one unit of BSF-2/HGF activity. This unitage is used in the present experiments.

### Example 7. Growth inhibition and differentiation of myeloleukemic cells

IFN-β2 is also active in growth inhibition and differentiation of myeloleukemic cells. Murine myeloleukemic MI cells and human histiocytic lymphoma U937 cells were grown in RPMI 1640 with 10% fetal calf serum (FCS). The cells were seeded at 10⁵ per ml in wells of 12-well Costar plates. Pure E. coli IFN-β2 was added at 0.1-75 ng/ml and the cultures were observed for 4-6 days. Cells were counted and stained for Giemsa and for non-specific esterase using the α-naphtyl acetate esterase kit 91-A of Sigma (St. Louis, MO). Lysozyme activity was measured in 0.5% Triton-X100 cell extracts by a turbidimetric assay of the lysis of Micrococcus lysodeikticus (Sigma Co.), the assay being calibrated with egg-white lysozyme as described (Weisinger, G. and Sachs, L. (1983) EMBO J. 3, pp. 2103-2107). The (2′-5′) oligo-A synthetase activity was assayed in Nonidet-P40 cell extracts as described (Resnitzky et al. (1986) Cell 46, pp. 31-40).

**Table 4**

| Effect of IFN-β2/IL-6 on myeloleukemic M1 cell growth | | | | | |
|---|---|---|---|---|---|
| IFN-β2 BSF U/ml | Cell number x 10⁻⁵ | | | | |
| | Day 0 | Day 1 | Day 4 | Day 5 | Day 6 |
| 0 | 1 | 2.3 | 19.0 | 25.0 | 35.0 |
| 25 | 1 | 2.0 | 3.5 | 0.9 | 0.7 |
| 50 | 1 | 1.4 | 2.3 | 0.8 | 1.2 |
| Recombinant E. coli IFN-β2 purified by Mono-S FPLC (Example 5C) | | | | | |

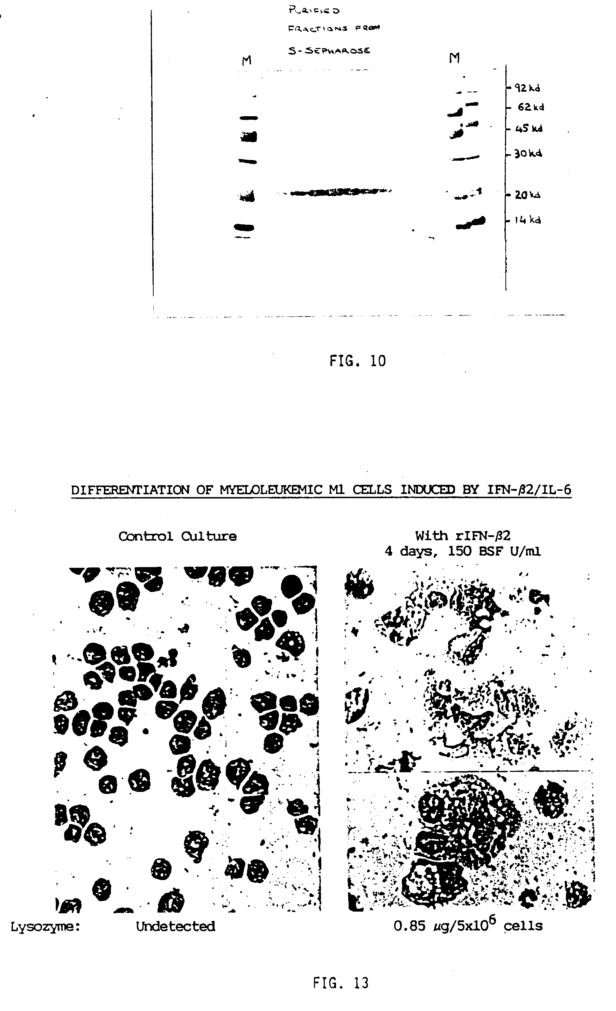

Without addition, the M1 cells grew without adhering to the dish and showed typical myeloblastic morphology. In contrast, after 4 days of culture with IFN-β2, the cells were adherent and showed dramatic morphological changes (Figure 13). About 60% of the cells acquired macrophage-like morphology, the rest showing various degrees of maturation. Cytoplasms were enlarged, contained vacuoles and acquired typical foamy appearance. Nuclei were eccentric, less round and contrasted and had less prominent nucleoli. Viable cell counting showed that while the control culture grew for 6 days, the M1 cells treated by 50 U/ml IFN-β2 underwent 2-3 divisions and growth was arrested (Table 4). At day 4 after seeding, less than 1 U/ml IFN-β2 (expressed in plasmacytoma growth units) was sufficient to cause a 50% decrease in M1 cell number. The growth-arrest effect was maximal about 30U/ml IFN-β2 (15 ng/ml). Even with the chemically purified rIFN-β2, this concentration corresponds to no more than 2.5 pg/ml LPS which had no effect on the M1 cells. Growth inhibition and differentiation of M1 cells was observed when IFN-β2 was added with 5 µg/ml polymyxin B, further excluding any role of LPS traces. As a biochemical marker of differentiation we measured lysozyme activity in extracts of 5 x 10⁶ M1 cells cultured 4 days with 30 U/ml IFN-β2. Lysozyme was undetectable in the control M1 cultures. Treatment with IFN-β2 induced lysozyme to levels of 0.85 µg lysozyme equivalent per 5 x 10⁶ cells. Phagocytic activity on latex beads was also observed in the differentiated M1 cells.

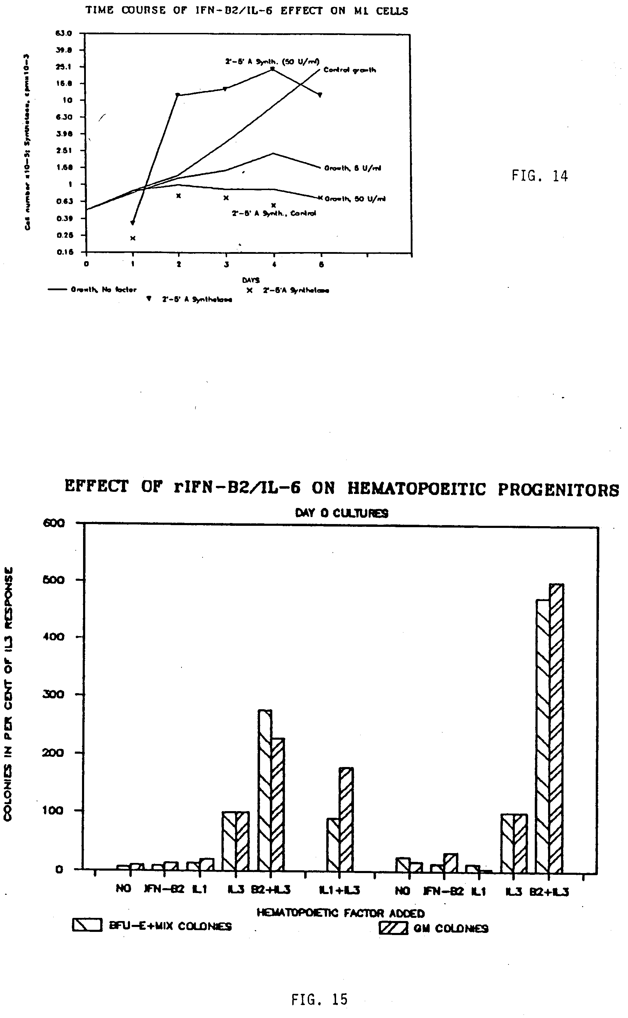

In another experiment, addition of IFN-β2/IL-6 to cultures of M1 cells arrested the growth of the cells after 24 hours (Fig. 14) and induced their differentiation into macrophages. At 24 hours, the cells already showed cytoplasmic enlargement with acentric nuclei and after 3-4 days acquired typical macrophage morphology demonstrated by increase in lysozyme phagocytotic activity and increase in Mac 1 antigen. The 50% growth inhibition of M1 cells was observed with about 0.5 ng/ml of rIFN-β2/IL-6, less than what is required for stimulation of plasmacytoma T1165 cells. The effect of IFN-β2/IL-6 was more rapid than that of the combination of IL-1 (10 U/ml) and TNF (10³ U/ml) which produced growth arrest only after 48 hours. These cytokines which also cause M1 differentiation are known inducers of IFN-β2/IL-6. The growth-arrest by IFN-β2/IL-6 was fully neutralized by monoclonal antibody 34.

**Table 5**

| Effect of IFN-β2/IL-6 on histiocytic lymphoma U937 cell growth and differentiation | | | |
|---|---|---|---|
| Expt | IFN-β2 BSF U/ml | Cell number x 10⁻⁵ | Esterase positive Cells, per cent |
| 1. | 0 | 14.0 (100).0 | 4 |
| | 100 | 10.0 ( 71) | 24 |
| 2. | 0 | 26.7 (100) | N.D. |
| | 150 | 23.5 ( 88) | " |
| | 1500 | 14.5 ( 54) | " |
| Cells treated for 5 days with or without rIFN-β2 purified on Mono-S FPLC. (Example 5C) | | | |

Human histiocytic lymphoma U937 cells can be induced to differentiate by phorbol esters and Vitamin D3, partially by IFN-gamma and other yet unidentified cytokines. We examined the effect of 100 HGF U/ml IFN-β2 addition on U937 cultures. After 4-5 days, about 25% of the cells showed monocytic/macrophage morphology and there was a 30% reduction in cell growth (Table 5). The cells were stained for α-naphtyl acetate esterase as a biochemical marker of differentiation not induced by IFN-gamma. About a fourth of the cells in the IFN-β2 treated culture were strongly positive for the non-specific esterase, whereas few positive cells were observed in the non-treated culture (Table 5) or in mock treated culture (not shown). With higher amounts of the pure rIFN-β2 preparations, growth inhibition (Table 5) and partial morphological changes, such as cytoplasmic enlargement and nucleus indentation, were more pronounced. However, we found that when added together with IFN-gamma, the effect of low dose IFN-β2 was significantly potentiated (Table 6). Under these conditions, cell growth was reduced and most of the cells showed cytoplasmic enlargement, changes in nuclear shape and nucleoli reduction, although monocytic differentiation was still incomplete. Thus we found that the combination of IFN-gamma 100 U/ml and IFN-β2 (1-10 HGF U/ml) has a synergistic effect and triggers growth arrest and differentiation. In optimal conditions, IFN-gamma alone reduced growth (after 6 days) by 10%, IFN-β2 alone by 25% and the combination IFN-gamma and IFN-β2 by 90%.

**Table 6**

| Synergistic effects of IFN-β2/IL-6 and IFN-gamma on histiocytic lymphoma U937 cells | | | |
|---|---|---|---|
| IFN-β2 BSF U/ml | IFN-gamma U/ml | Cell number x 10⁻⁵ | (2'-5') A Synthetase Activity ³²P-A2'pA, cpm |
| 0 | 0 | 20 (100) | 110 |
| 15 | 0 | 14 ( 70) | 310 |
| 0 | 100 | 12 ( 60) | 940 |
| 15 | 100 | 8.5 ( 42) | 4,000 |
| Cells treated for 6 days with or without rIFN-β2 immunoaffinity purified. | | | |

The addition of IFN-gamma also strongly potentiated the induction of (2′-5′) Oligo A synthetase by IFN-β2 (Table 6) suggesting that the two cytokines cooperate to initiate the differentiation process, although other additions may be required to see complete differentiation of the type seen with the M1 cells.

IFN-β2 activity on fresh leukemic cells of acute myelogenous leukemia (AML) was also studied. Peripheral blood mononuclear cells from AML patients incubated 5 days with rIFN-β2/IL-6 showed a decrease in the percentage of blast cells (from 20-30% in control cultures to 6-12% with IFN-β2/IL-6) with an increase in myelocytic forms at various stages of differentiation and in the ratio of myelomonocytes to blast cells. The results with two AML patients are shown in Table 7. GM-CSF was also tested (comparison). IFN-β2/IL-6, therefore acts also on fresh leukemic cells and such tests may be useful to foresee the therapeutic value of the cytokine in AML.

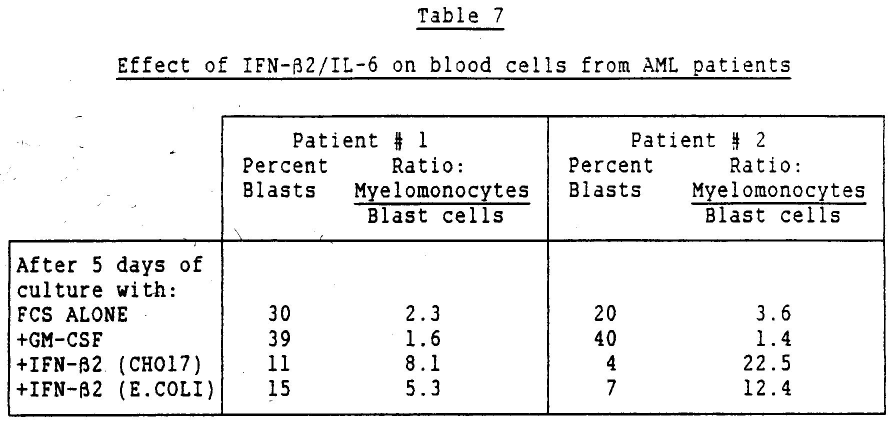

### Example 8. Hematopoietic effects on normal bone marrow cultures

Monocyte and T-cell depleted human bone marrow cells treated by 4-hydroperoxy cyclophosphamide (4-HC) (100µg/ml, 30 min.) to eliminate committed progenitors of CFU-mix (colony-forming unit - granulocyte, monocyte, erythroid, megakaryocytes), CFU-GM (colony-forming unit - granulocyte, monocyte) and BFU-E (burst-forming unit - erythroid) colonies, were used to study the effects of rIFN-β2/IL-6 on the early steps in hematopoietic differentiation. When added at the time cells were plated on methylcellulose (10⁵ cells/ml), IFN-β2/IL-6 by itself could not support the growth of colonies, indicating it does not function as a growth-promoting CSF (colony stimulating factor) (Fig. 15). However, IFN-β2/IL-6 markedly increased the ability of IL-3 to cause formation of colonies with mixed (CFU-GEMM) and erythroid (BFU-E) as well as granulocytic monocytic (CGU-GM) phenotypes. In this action IFN-β2/IL-6 appears more potent than IL-1 (Fig. 15). In a two-stage assay, where IFN-β2/IL-6 was added in liquid cultures one week before the cells were plated in methylcellulose with a full supplement of CSF, an increase in the number of progenitor cells able to respond to CSF was produced by IFN-β2/IL-6 alone (Fig. 15, right). This increase was only slightly lower than that caused by IL-3 and the two factors seem to work independently in this first stage of the assay. In Fig. 15, colonies were counted after 15 days and classified as CFU-mix, CFU-GM and BFU-E Left half: Day 0 cultures with no addition (10% fetal calf serum and erythropoietin), and with the addition of 10 HGF U/ml rIFN-β2/IL-6, 2 U/ml rIL-1, 10 U/ml rIL-3, IFN-β2/IL-6+IL-3 or IL-1+IL-3. Right half: Cells were first incubated for one week in liquid cultures with no addition and in the presence of rIFN-β2/IL-6, rIL-1, rIL-3, IFN-β2/IL-6+IL-3 or IL-1+IL-3. The cells were then plated as above in methylcellulose for 15 days with PHA-induced leucocyte conditioned medium (containing all CSF) and colonies counted.

The stimulation of mixed colonies from normal bone marrow progenitor cells is significant enough to warrant the use of rIFN-β2/IL-6 in bone marrow transplants.

### Example 9. Induction of Complement Factor B in fibroblasts

The induction of Complement Factor B in human diploid skin fibroblasts GM8399 by IFN-β2 was studied. When used alone, the immunopurified IFN-β2 induced the secretion of Complement Factor B but the effect was again strongly potentiated by IFN-gamma. This effect is of importance since Complement Factor B is an essential component of the alternative pathway of complement which kills bacteria and parasites without need for antibodies. A local increase in resistance to such infectious agents can be expected in response to the IFN-β2 - IFN-gamma combination. A biological assay for Factor B activity showed increase in complement activity for cell lysis. The synergistic effect of IFN-gamma and IFN-β2 suggests that this combination may prove very attractive.

### Pharmaceutical Compositions

Human IFN-β2 may be used for the treatment
of breast cancer, leukemia, e.g. acute myelogenous leukemia, infectious diseases caused by bacteria or parasite and in bone marrow transplants. It may be used alone or in combination with other cytokines, in particular with IFN-gamma for the treatment of infectious diseases and certain types of leukemia. The active IFN-β2 may be administered by any route appropriate to the condition being treated. It may be formulated with one or more pharmaceutically acceptable carriers and systematically administered either parenterally, intravenously or subcutaneously, or enterally, e.g. in the form of a tablet, capsules, etc.

The amount of active ingredient to be administered will be determined by the physician and will depend upon several factors, e.g. the severity of the condition being treated, weight, age and general condition of the patient, the route of administration chosen and the specific activity of the active IFN-β2. Daily dosages could be in the range of about 5 micrograms to about 800 micrograms, preferably within the range of 10-100 micrograms per day.

The pharmaceutical compositions may conveniently be presented in unit dosage form and may be prepared by any of the methods well known in the art.

## Claims (Claims for the following Contracting State(s): AT, BE, CH, LI, DE, FR, GB, GR, IT, LU, NL, SE)

1. A monoclonal antibody capable of specifically binding to natural human interferon-β2 (IFN-β2) as well as to recombinant human IFN-β2 expressed by mammalian cells and recombinant human IFN-β2 expressed by bacterial cells, capable of inhibiting the biological activity of human IFN-β2 when bound thereto, and capable of releasing IFN-β2 bound thereto upon elution with 50 mM citric acid.

2. A monoclonal antibody in accordance with claim 1, of class IgG1.

3. A monoclonal antibody in accordance with claim 1, which is produced by a hybridoma cell obtained by fusion of myeloma cells with spleen cells from a mouse previously immunized with recombinant human IFN-β2 expressed by bacterial cells or with a recombinant fusion protein including human IFN-β2 expressed by bacterial cells, said hybridoma cell being selected for the production of a monoclonal antibody which binds to recombinant human IFN-β2 expressed by mammalian cells.

4. A monoclonal antibody in accordance with claim 3, wherein the myeloma cells are murine myeloma cells and the spleen cells are from a mouse immunized with a recombinant fusion protein including human IFN-β2.

5. A monoclonal antibody in accordance with claim 4, wherein the fusion protein is Protein A-IFN-β2.

6. A monoclonal antibody expressible by hybridoma cell line CNCM I-813 (HB2 34-1).

7. A hybridoma cell line capable of expressing a monoclonal antibody in accordance with claim 1.

8. A hybridoma cell line in accordance with claim 7 obtained by fusion of myeloma cells with spleen cells from a mouse previously immunized with recombinant human IFN-β2 expressed by bacterial cells or with a recombinant fusion protein including human IFN-β2 expressed by bacterial cells, said hybridoma cell being selected for the production of a monoclonal antibody which binds to recombinant human IFN-β2 expressed by mammalian cells.

9. A hybridoma cell line according to claim 8, obtained by fusion of murine myeloma cells with spleen cells from a mouse previously immunized with a recombinant fusion protein including human IFN-β2.

10. A hybridoma cell line according to claim 9, wherein the fusion protein is Protein A-IFN-β2.

11. The hybridoma cell line CNCM I-813 (No.34-1).

12. A method for obtaining a monoclonal antibody in accordance with claim 1, which comprises:
(a) immunizing mice with human IFN-β2 or with a fusion protein comprising human IFN-β2, wherein the source of said IFN-β2 or fusion protein is one of the sources selected from the group consisting of natural protein, recombinant protein produced in bacterial cells, and recombinant protein produced in mammalian cells;
(b) fusing the spleen cells from said mice with murine myeloma cells in the presence of a suitable fusion promoter;
(c) testing the supernatant of the cultured fused cells for the presence of the desired monoclonal antibodies with human IFN-β2 from a different one of said sources than that of the IFN-β2 used in step (a) above; and
(d) selecting and cloning the hybridoma cell line producing the desired monoclonal antibodies, and either (i) culturing the selected hybridoma cell line in a suitable growth medium and recovering the desired monoclonal antibodies from the supernatant, or (ii) injecting the selected hybridoma cell line into mice and recovering the desired monoclonal antibodies from the ascitic fluid of said mice.

13. A method in accordance with claim 12, wherein in step (a) the mice are immunized with recombinant human IFN-β2 expressed by bacterial cells or with a recombinant fusion protein including human IFN-β2 expressed by bacterial cells and the hybridoma supernatants of step (c) are tested with recombinant human IFN-β2 expressed by mammalian cells.

14. A method according to claim 12, wherein in step (a) the mice are immunized with Protein A-IFN-β2 expressed by *E. coli* and the hybridoma supernatants of step (c) are tested with IFN-β2 expressed by CHO cells.

15. A method for producing anti-IFN-β2 monoclonal antibodies which comprises culturing hybridoma CNCM I-813 in a suitable growth medium and recovering the monoclonal antibodies from the supernatant of said hybridoma.

16. A method for producing anti-IFN-β2 monoclonal antibodies which comprises injecting hybridoma CNCM I-813 in a mouse and recovering the monoclonal antibodies from the ascitic fluid of said mouse.

17. A method for obtaining a hybridoma cell line in accordance with claim 7, comprising:
(a) immunizing mice with human IFN-β2 or with a fusion protein comprising human IFN-β2, wherein the source of said IFN-β2 or fusion protein is one of the sources selected from the group consisting of natural protein, recombinant protein produced in bacterial cells, and recombinant protein produced in mammalian cells;
(b) fusing the spleen cells from said mice with murine myeloma cells in the presence of a suitable fusion promoter;
(c) testing the supernatant of the cultured fused cells for the presence of the desired monoclonal antibodies with human IFN-β2 from a different one of said sources than that of the IFN-β2 used in step (a) above; and
(d) selecting and cloning the hybridoma cell line producing the desired monoclonal antibodies.

18. A method according to claim 17, wherein in step (a) the mice are immunized with Protein A-IFN-β2 expressed by *E. coli* and the hybridoma supernatants of step (c) are tested with IFN-β2 expressed by CHO cells.

19. A method for the immunopurification of human biologically active IFN-β2 comprising passing a sample containing human IFN-β2 through an immunoadsorbent column comprising a monoclonal antibody in accordance with claim 1 bound to a solid phase support washing the column, and eluting IFN-β2 from the column.

## Claims (Claims for the following Contracting State(s): ES)

1. A method for producing a hybridoma cell line capable of producing a monoclonal antibody, which monoclonal antibody is capable of specifically binding to natural human interferon-β2 (IFN-β2) as well as to recombinant human IFN-β2 expressed by mammalian cells and recombinant human IFN-β2 expressed by bacterial cells, capable of inhibiting the biological activity of human IFN-β2 when bound thereto, and capable of releasing IFN-β2 bound thereto upon elution with 50 mM citric acid,
wherein myeloma cells are fused with spleen cells from a mouse previously immunized with recombinant human IFN-β2 expressed by bacterial cells or with a recombinant fusion protein including human IFN-β2 expressed by bacterial cells and a hybridoma cell line is selected for the production of a monoclonal antibody which binds to recombinant human IFN-β2 expressed by mammalian cells.

2. The method in accordance with claim 1, wherein the myeloma cells are murine myeloma cells and the spleen cells are from a mouse immunized with a recombinant fusion protein including human IFN-β2.

3. The method in accordance with claim 2, wherein the fusion protein is Protein A-IFN-β2.

4. The method according to claim 1 comprising:
(a) immunizing mice with human IFN-β2 or with a fusion protein comprising human IFN-β2, wherein the source of said IFN-β2 or fusion protein is one of the sources selected from the group consisting of natural protein, recombinant protein produced in bacterial cells, and recombinant protein produced in mammalian cells;
(b) fusing the spleen cells from said mice with murine myeloma cells in the presence of a suitable fusion promoter;
(c) testing the supernatant of the cultured fused cells for the presence of the desired monoclonal antibodies with human IFN-β2 from a different one of said sources than that of the IFN-β2 used in step (a) above; and
(d) selecting and cloning the hybridoma cell line producing the desired monoclonal antibodies.

5. The method according to claim 4, wherein in step (a) the mice are immunized with Protein A-IFN-β2 expressed by *E. coli* and the hybridoma supernatants of step (c) are tested with IFN-β2 expressed by CHO cells.

6. The hybridoma cell line CNCM I-813 (No.34-1).

7. A method for obtaining a monoclonal antibody capable of specifically binding to natural human interferon-β2 (IFN-β2) as well as to recombinant human IFN-β2 expressed by mammalian cells and recombinant human IFN-β2 expressed by bacterial cells, capable of inhibiting the biological activity of human IFN-β2 when bound thereto, and capable of releasing IFN-β2 bound thereto upon elution with 50 mM citric acid which comprises:
(a) immunizing mice with human IFN-β2 or with a fusion protein comprising human IFN-β2, wherein the source of said IFN-β2 or fusion protein is one of the sources selected from the group consisting of natural protein, recombinant protein produced in bacterial cells, and recombinant protein produced in mammalian cells;
(b) fusing the spleen cells from said mice with murine myeloma cells in the presence of a suitable fusion promoter;
(c) testing the supernatant of the cultured fused cells for the presence of the desired monoclonal antibodies with human IFN-β2 from a different one of said sources than that of the IFN-β2 used in step (a) above; and
(d) selecting and cloning the hybridoma cell line producing the desired monoclonal antibodies, and either (i) culturing the selected hybridoma cell line in a suitable growth medium and recovering the desired monoclonal antibodies from the supernatant, or (ii) injecting the selected hybridoma cell line into mice and recovering the desired monoclonal antibodies from the ascitic fluid of said mice.

8. The method according to claim 7, wherein the antibody is of class IgG1.

9. A method in accordance with claim 7, wherein in step (a) the mice are immunized with recombinant human IFN-β2 expressed by bacterial cells or with a recombinant fusion protein including human IFN-β2 expressed by bacterial cells and the hybridoma supernatants of step (c) are tested with recombinant human IFN-β2 expressed by mammalian cells.

10. A method according to claim 7, wherein in step (a) the mice are immunized with Protein A-IFN-β2 expressed by *E. coli* and the hybridoma supernatants of step (c) are tested with IFN-β2 expressed by CHO cells.

11. A method for producing anti-IFN-β2 monoclonal antibodies which comprises culturing hybridoma CNCM I-813 in a suitable growth medium and recovering the monoclonal antibodies from the supernatant of said hybridoma.

12. A monoclonal antibody expressible by hybridoma cell line CNCM I-813 (HB2 34-1).

13. A method for producing anti-IFN-β2 monoclonal antibodies which comprises injecting hybridoma CNCM I-813 in a mouse and recovering the monoclonal antibodies from the ascitic fluid of said mouse.

14. A method for the immunopurification of human biologically active IFN-β2 comprising passing a sample containing human IFN-β2 through an immunoadsorbent column comprising a monoclonal antibody produced in accordance with claim 7 bound to a solid phase support washing the column, and eluting IFN-β2 from the column.

## Patentansprüche (Patentansprüche für folgende(n) Vertragsstaat(en): AT, BE, CH, LI, DE, FR, GB, GR, IT, LU, NL, SE)

1. Monoclonaler Antikörper, der in der Lage ist sowohl spezifisch an natürliches, menschliches, Interferon-β2 (IFN-β2) als auch an rekombinantes, menschliches, in Säugerzellen exprimiertes IFN-β2 und an rekombinantes, menschliches, in Bakterienzellen exprimiertes IFN-β2, zu binden, wobei der monoclonale Antikörper in der Lage ist die biologische Aktivität von menschlichem IFN-β2 zu inhibieren, wenn er an dieses gebunden ist und er in der Lage ist durch Elution mit 50 mM Zitronensäure an ihn gebundenes IFN-β2 freizusetzen.

2. Monoclonaler Antikörper nach Anspruch 1, der Klasse IgG1.

3. Monoclonaler Antikörper nach Anspruch 1, der hergestellt wird von einer Hybridomzelle erhalten durch Fusion von Myelomzellen mit Milzzellen einer Maus, die vorher mit rekombinantem, menschlichem, in Bakterienzellen exprimiertem IFN-β2 oder mit einem rekombinanten Fusionsprotein einschließlich menschlichem, in Bakterienzellen exprimiertem IFN-β2 immunisiert wurde, wobei die Hybridomzelle zum Herstellen eines monoclonalen Antikörpers, der an rekombinantes, menschliches, in Säugerzellen exprimiertes IFN-β2 bindet, ausgewählt ist.

4. Monoclonaler Antikörper nach Anspruch 3, wobei die Myelomzellen von Mäusen oder Ratten abstammende Myelomzellen sind, und die Milzzellen von einer Maus stammen, die mit einem rekombinanten Fusionsprotein, einschließlich menschlichem IFN-β2, immunisiert wurde.

5. Monoclonaler Antikörper nach Anspruch 4, wobei das Fusionsprotein Protein A-IFN-β2 ist.

6. Monoclonaler Antikörper exprimierbar durch die Hybridomzellinie CNCM I-813 (HB2 34-1).

7. Hybridomzellinie, die in der Lage ist einen monoclonalen Antikörper nach Anspruch 1 zu exprimieren.

8. Hybridomzellinie nach Anspruch 7, erhalten durch Fusion von Myelomzellen mit Milzzellen von einer Maus, die vorher immunisiert wurde mit rekombinantem, menschlichem, in Bakterienzellen exprimiertem IFN-β2, oder mit einem rekombinanten Fusionsprotein, einschließlich menschlichem, in Bakterienzellen exprimiertem IFN-β2, wobei die Hybridomzelle zum Herstellen eines monoclonalen Antikörpers ausgewählt ist, der rekombinantes, menschliches, in Säugerzellen exprimiertes IFN-β2 bindet.

9. Hybridomzellinie nach Anspruch 8, erhalten durch Fusion von von Mäusen oder Ratten abstammenden Myelomzellen mit Milzzellen einer Maus, die vorher mit einem rekombinanten Fusionsprotein, einschließlich menschlichem IFN-β2, immunisiert wurde.

10. Hybridomzellinie nach Anspruch 9, wobei das Fusionsprotein Protein A-IFN-β2 ist.

11. Hybridomzellinie CNCM I-813(Nr. 34-1).

12. Verfahren zum Erhalten eines monoclonalen Antikörpers nach Anspruch 1, das umfaßt:
(a) das Immunisieren von Mäusen mit menschlichem IFN-β2 oder mit einem menschliches IFN-β2 umfassenden Fusionsprotein, wobei die Quelle des IFN-β2 oder des Fusionsproteins eine der Quellen ausgewählt aus der Gruppe bestehend aus natürlichem Protein, rekombinantem, in Bakterienzellen hergestelltem Protein und rekombinantem, in Säugerzellen hergestelltem Protein, ist;
(b) das Fusionieren der Milzzellen der Maus mit von Mäusen oder Ratten abstammenden Myelomzellen in der Gegenwart eines geeigneten Fusionspromoters;
(c) das Testen des Überstandes der kultivierten fusionierten Zellen auf die Anwesenheit der gewünschten monoclonalen Antikörper mit menschlichem IFN-β2 aus einer anderen Quelle als der Quelle des IFN-β2, welches oben in Schritt (a) benutzt wurde; und
(d) das Auswählen und Klonieren der Hybridomzellinie, die die gewünschten monoclonalen Antikörper herstellt und entweder (i) Kultivieren der ausgewählten Hybridomzellinie in einem geeigneten Wachstumsmedium und Wiedergewinnen der gewünschten monoclonalen Antikörper aus dem Überstand, oder (ii) Injizieren der ausgewählten Hybridomzellinie in Mäuse und Wiedergewinnen der gewünschten monoclonalen Antikörper aus der Bauchwasserflüssigkeit der Mäuse.

13. Verfahren nach Anspruch 12, wobei in Schritt (a) die Mäuse immunisiert werden mit rekombinantem, menschlichem, in Bakterienzellen exprimiertem IFN-β2 oder mit einem rekombinanten Fusionsprotein, einschließlich menschlichem, in Bakterienzellen exprimiertem IFN-β2 und die Hybridomüberstände aus Schritt (c) mit rekombinantem, menschlichem, in Säugerzellen exprimiertem IFN-β2 getestet werden.

14. Verfahren nach Anspruch 12, wobei in Schritt (a) die Mäuse mit in E. coli exprimiertem Protein A-IFN-β2 immunisiert werden und die Hybridomüberstände aus Schritt (c) mit in CHO-Zellen exprimiertem IFN-β2 getestet werden.

15. Verfahren zum Herstellen von anti-IFN-β2 monoclonalen Antikörpern, welches das Kultivieren von Hybridom CNCM I-813 in einem geeigneten Wachstumsmedium und das Wiedergewinnen der monoclonalen Antikörper aus dem Überstand des Hybridoms umfaßt.

16. Verfahren zum Herstellen von anti-IFN-β2 monoclonalen Antikörpern, welches das Injizieren von Hybridom CNCM I-813 in eine Maus und das Wiedergewinnen der monoclonalen Antikörper aus der Bauchwasserflüssigkeit der Maus umfaßt.

17. Verfahren zum Erhalten einer Hybridomzellinie nach Anspruch 7, umfassend:
(a) das Immunisieren von Mäusen mit menschlichem IFN-β2 oder mit einem menschliches IFN-β2 umfassenden Fusionsprotein, wobei die Quelle des IFN-β2 oder des Fusionsproteins eine der Quellen ausgewählt aus der Gruppe bestehend aus natürlichem Protein, rekombinantem, in Bakterienzellen hergestelltem Protein und rekombinantem, in Säugerzellen hergestelltem Protein, ist;
(b) das Fusionieren der Milzzellen der Maus mit von Mäusen oder Ratten abstammenden Myelomzellen in der Gegenwart eines geeigneten Fusionspromoters;
(c) das Testen des Überstandes der kultivierten fusionierten Zellen auf die Anwesenheit der gewünschten monoclonalen Antikörper mit menschlichem IFN-β2 aus einer anderen Quelle als der Quelle des IFN-β2 welches oben in Schritt (a) benutzt wurde; und
(d) das Auswählen und Klonieren der Hybridomzellinie, die die gewünschten monoclonalen Antikörper herstellt.

18. Verfahren nach Anspruch 17, wobei in Schritt (a) die Mäuse mit in E. coli exprimiertem Protein A-IFN-β2 immunisiert werden und die Hybridomüberstände aus Schritt (c) mit in CHO-Zellen exprimiertem IFN-β2 getestet werden.

19. Verfahren zum Immunreinigen von menschlichem, biologisch aktivem IFN-β2, umfassend das Leiten einer menschliches IFN-β2 enthaltenden Probe durch eine immunabsorbierende Säule, die einen monoclonalen Antikörper nach Anspruch 1 umfaßt, der an einen Festträger gebunden ist,
das Waschen der Säule und
das Eluieren des IFN-β2 von der Säule.

## Patentansprüche (Patentansprüche für folgende(n) Vertragsstaat(en): ES)

1. Verfahren zum Herstellen einer Hybridomzellinie, die in der Lage ist einen monoclonalen Antikörper herzustellen, welcher in der Lage ist spezifisch an natürliches, menschliches Interferon-β2 (IFN-β2) zu binden und ebenso an rekombinantes, menschliches, in Säugerzellen exprimiertes IFN-β2 und rekombinantes, menschliches, in Bakterienzellen exprimiertes IFN-β2 zu binden, wobei der monoclonale Antikörper in der Lage ist die biologische Aktivität des menschlichen IFN-β2 zu inhibieren, wenn er an dieses gebunden ist und er in der Lage ist durch Elution mit 50 mM Zitronensäure an ihn gebundenes IFN-β2 freizusetzen,
wobei Myelomzellen mit Milzzellen einer Maus fusioniert werden, die vorher mit rekombinantem, menschlichem, in Bakterienzellen exprimiertem IFN-β2 oder mit einem rekombinanten Fusionsprotein, einschließlich menschlichem, in Bakterienzellen exprimiertem IFN-β2, immunisiert wurde und eine Hybridomzellinie ausgewählt wird zum Herstellen eines monoclonalen Antikörpers der an rekombinantes, menschliches, in Säugerzellen exprimiertes IFN-β2 bindet.

2. Verfahren nach Anspruch 1, wobei die Myelomzellen von Mäusen oder Ratten abstammenden Myelomzellen sind und die Milzzellen von einer Maus stammen, die mit rekombinantem Fusionsprotein einschließlich menschlichem IFN-β2 immunisiert wurde.

3. Verfahren nach Anspruch 2, wobei das Fusionsprotein Protein A-IFN-β2 ist.

4. Verfahren nach Anspruch 1 umfassend:
(a) das Immunisieren von Mäusen mit menschlichem IFN-β2 oder mit einem menschliches IFN-β2 umfassenden Fusionsprotein, wobei die Quelle des IFN-β2 oder des Fusionsproteins eine der Quellen ausgewählt aus der Gruppe bestehend aus natürlichem Protein, rekombinantem, in Bakterienzellen hergestelltem Protein und rekombinantem in Säugerzellen hergestelltem Protein, ist;
(b) das Fusionieren der Milzzellen der Maus mit von Mäusen oder Ratten abstammenden Myelomzellen in der Gegenwart eines geeigneten Fusionspromoters;
(c) das Testen des Überstandes der kultivierten fusionierten Zellen auf die Anwesenheit der gewünschten monoclonalen Antikörper mit menschlichem IFN-β2 aus einer anderen Quelle als der Quelle des IFN-β2, welches oben in Schritt (a) benutzt wurde; und
(d) das Auswählen und Klonieren der Hybridomzellinie, die die gewünschten monoclonalen Antikörper herstellt.

5. Verfahren nach Anspruch 4, wobei in Schritt (a) die Mäuse mit in E. coli exprimiertem Protein A-IFN-β2 immunisiert werden und die Hybridomüberstände aus Schritt (c) mit in CHO-Zellen exprimiertem IFN-β2 getestet werden.

6. Hybridomzellinie CNCM I-813 (Nr. 34-1).

7. Verfahren zum Erhalten eines monoclonaler Antikörper, der in der Lage ist sowohl spezifisch an natürliches menschliches Interferon-β2 (IFN-β2) als auch an rekombinantes, menschliches, in Säugerzellen exprimiertes IFN-β2, zu binden und an rekombinantes, menschliches, in Bakterienzellen exprimiertes IFN-β2, zu binden, wobei der monoclonale Antikörper in der Lage ist die biologische Aktivität von menschlichem IFN-β2 zu inhibieren, wenn er an dieses gebunden ist und er in der Lage ist durch Elution mit 50 mM Zitronensäure an ihnen gebundenes IFN-β2 freizusetzen, welches umfaßt:
(a) das Immunisieren von Mäusen mit menschlichem IFN-β2 oder mit einem menschliches IFN-β2 umfassenden Fusionsprotein, wobei die Quelle des IFN-β2 oder des Fusionsproteins eine der Quellen ausgewählt aus der Gruppe bestehend aus natürlichem Protein, rekombinantem, in Bakterienzellen hergestelltem Protein und rekombinantem in Säugerzellen hergestelltem Protein, ist;
(b) das Fusionieren der Milzzellen der Maus mit von Mäusen oder Ratten abstammenden Myelomzellen in der Gegenwart eines geeigneten Fusionspromoters;
(c) das Testen des Überstandes der kultivierten fusionierten Zellen auf die Anwesenheit der gewünschten monoclonalen Antikörper mit menschlichem IFN-β2 aus einer anderen Quelle als der Quelle des IFN-β2, welches oben in Schritt (a) benutzt wurde; und
(d) das Auswählen und Klonieren der Hybridomzellinie, die die gewünschten monoclonalen Antikörper herstellt und entweder (i) Kultivieren der ausgewählten Hybridomzellinie in einem geeigneten Wachstumsmedium und Wiedergewinnen der gewünschten monoclonalen Antikörper aus dem Überstand, oder (ii) Injizieren der ausgewählten Hybridomzellinie in Mäuse und Wiedergewinnen der gewünschten monoclonalen Antikörper aus der Bauchwasserflüssigkeit der Mäuse.

8. Verfahren nach Anspruch 7, wobei der Antikörper der Klasse IgG1 angehört.

9. Verfahren nach Anspruch 7, wobei in Schritt (a) die Mäuse immunisiert werden mit rekombinantem, menschlichem, in Bakterienzellen exprimiertem IFN-β2 oder mit einem rekombinanten Fusionsprotein, einschließlich menschlichem, in Bakterienzellen exprimiertem IFN-β2 und die Hybridomüberstände aus Schritt (c) mit rekombinantem, menschlichem, in Säugerzellen exprimiertem IFN-β2 getestet werden.

10. Verfahren nach Anspruch 7, wobei in Schritt (a) die Mäuse mit in E. coli exprimiertem Protein A-IFN-β2 immunisiert werden und die Hybridomüberstände aus Schritt (c) mit in CHO-Zellen exprimiertem IFN-β2 getestet werden.

11. Verfahren zum Herstellen von anti-IFN-β2 monoclonalen Antikörpern, welches das Kultivieren von Hybridom CNCM I-813 in einem geeigneten Wachstumsmedium und das Wiedergewinnen der monoclonalen Antikörper aus dem Überstand des Hybridoms umfaßt.

12. Monoclonaler Antikörper exprimierbar durch die Hybridomzellinie CNCM I-813 (HB2 34-1).

13. Verfahren zum Herstellen von anti-IFN-β2 monoclonalen Antikörpern, welches das Injizieren von Hybridom CNCM I-813 in eine Maus und das Wiedergewinnen der monoclonalen Antikörper aus der Bauchwasserflüssigkeit der Maus umfaßt.

14. Verfahren zum Immunreinigen von menschlichem biologisch aktivem IFN-β2, umfassend das Leiten einer menschliches IFN-β2 enthaltenden Probe durch eine immunabsorbierende Säule, die einen monoclonalen Antikörper nach Anspruch 1 umfaßt, der an einen Festträger gebunden ist,
das Waschen der Säule und
das Eluieren des IFN-β2 von der Säule.

## Revendications (Revendications pour l'(les) Etat(s) contractant(s) suivant(s): AT, BE, CH, LI, DE, FR, GB, GR, IT, LU, NL, SE)

1. Anticorps monoclonal capable de se lier spécifiquement à l'interféron-β2 humain naturel (IFN-β2) ainsi qu'à IFN-β2 humain recombiné exprimé par des cellules de mammifères et IFN-β2 humain recombiné exprimé par des cellules bactériennes, capable d'inhiber l'activité biologique de IFN-β2 humain lorsqu'il y est lié et capable de libérer IFN-β2 qui lui est lié lors d'une élution avec de l'acide citrique 50 mM.

2. Anticorps monoclonal selon la revendication 1, de la classe IgG1.

3. Anticorps monoclonal selon la revendication 1, qui est produit par une cellule hybridome obtenue par fusion de cellules de myélome avec des cellules de rate en provenance d'une souris préalablement immunisée avec IFN-β2 humain recombiné exprimé par des cellules bactériennes ou avec une protéine de fusion recombinée contenant IFN-β2 humain exprimé par des cellules bactériennes, ladite cellule hybridome étant sélectionnée pour la production d'un anticorps monoclonal qui se lie à IFN-β2 humain recombiné exprimé par des cellules de mammifères.

4. Anticorps monoclonal selon la revendication 3, dans lequel les cellules de myélome sont des cellules de myélome de souris et les cellules de rate proviennent d'une souris immunisée avec une protéine de fusion recombinée contenant IFN-β2 humain.

5. Anticorps monoclonal selon la revendication 4, dans lequel la protéine de fusion est la Protéine A-IFN-β2.

6. Anticorps monoclonal pouvant être exprimé par la lignée de cellules hybridomes CNCM I-813 (HB2 34-1).

7. Lignée de cellules hybridomes capable d'exprimer un anticorps monoclonal selon la revendication 1.

8. Lignée de cellules hybridomes selon la revendication 7, obtenue par fusion de cellules de myélome avec des cellules de rate en provenance d'une souris préalablement immunisée par IFN-β2 humain recombiné exprimé par des cellules bactériennes ou par une protéine de fusion recombinée contenant IFN-β2 humain exprimé par des cellules bactériennes, ladite cellule hybridome étant sélectionnée pour la production d'un anticorps monoclonal qui se lie à IFN-β2 humain recombiné exprimé par des cellules de mammifères.

9. Lignée de cellules hybridomes selon la revendication 8, obtenue par fusion de cellules de myélome de souris avec des cellules de rate en provenance d'une souris préalablement immunisée par une protéine de fusion recombinée contenant IFN-β2 humain.

10. Lignée de cellules hybridomes selon la revendication 9, dans laquelle la protéine de fusion est la protéine A-IFN-β2.

11. Lignée de cellules hybridomes CNCM I-813 (n° 34-1).

12. Procédé pour obtenir un anticorps monoclonal selon la revendication 1, qui comprend les étapes suivantes :
(a) immuniser des souris avec IFN-β2 humain ou avec une protéine de fusion contenant IFN-β2 humain, la source dudit IFN-β2 ou de ladite protéine de fusion étant l'une des sources sélectionnées dans le groupe comprenant une protéine naturelle, une protéine recombinée produite dans des cellules bactériennes et une protéine recombinée produite dans des cellules de mammifères ;
(b) fusionner les cellules de rate en provenance de ladite souris avec des cellules de myélome de souris en la présence d'un promoteur de fusion approprié ;
(c) tester le surnageant des cellules fusionnées cultivées en ce qui concerne la présence des anticorps monoclonaux recherchés avec un IFN-β2 humain provenant d'une desdites sources différente de celle de l'IFN-β2 utilisé dans l'étape (a) ci-dessus, et
(d) sélectionner et cloner la lignée de cellules hybridomes produisant les anticorps monoclonaux recherchés ; et soit (i) cultiver la lignée de cellules hybridomes sélectionnée dans un milieu de croissance approprié et récupérer les anticorps monoclonaux désirés du surnageant, soit (ii) injecter la lignée de cellules hybridomes sélectionnée dans des souris et récupérer les anticorps monoclonaux recherchés du fluide ascitique des souris.

13. Procédé selon la revendication 12, dans lequel, dans l'étape (a), les souris sont immunisées avec IFN-β2 humain recombiné exprimé par des cellules bactériennes ou avec une protéine de fusion recombinée contenant IFN-β2 humain exprimé par des cellules bactériennes, et les surnageants des hybridomes de l'étape (c) sont testés avec IFN-β2 humain recombiné exprimé par des cellules de mammifères.

14. Procédé selon la revendication 12, dans lequel, dans l'étape (a), les souris sont immunisées avec la Protéine A-IFN-β2 exprimée par *E. coli* et les surnageants des hybridomes de l'étape (c) sont testés avec IFN-β2 exprimé par des cellules CHO.

15. Procédé pour produire des anticorps monoclonaux anti-IFN-β2, qui comprend les étapes suivantes : cultiver les hybridomes CNCM I-813 dans un milieu de croissance approprié et récupérer les anticorps monoclonaux du surnageant desdits hybridomes.

16. Procédé pour produire des anticorps monoclonaux anti-IFN-β2, qui comprend les étapes suivantes : injecter des hybridomes CNCM I-813 dans une souris et récupérer les anticorps monoclonaux du fluide ascitique de ladite souris.

17. Procédé pour obtenir une lignée de cellules hybridomes selon la revendication 7, comprenant les étapes suivantes :
(a) immuniser des souris avec IFN-β2 humain ou avec une protéine de fusion contenant IFN-β2 humain, la source dudit IFN-β2 ou de ladite protéine de fusion étant l'une des sources sélectionnées dans le groupe comprenant : une protéine naturelle, une protéine recombinée produite dans des cellules bactériennes et une protéine recombinée produite dans les cellules de mammifères ;
(b) fusionner les cellules de rate en provenance desdites souris avec des cellules de myélome de souris en la présence d'un promoteur de fusion approprié ;
(c) tester le surnageant des cellules fusionnées cultivées en ce qui concerne la présence des anticorps monoclonaux recherchés avec IFN-β2 humain en provenance de l'une desdites sources différente de celle de l'IFN-β2 utilisé dans l'étape (a) ci-dessus ; et
(d) sélectionner et cloner la lignée de cellules hybridomes produisant les anticorps monoclonaux

18. Procédé selon la revendication 17, dans lequel, dans l'étape (a), les souris sont immunisées avec la Protéine A-IFN-β2 exprimée par *E. coli* et les surnageants des hybridomes de l'étape (c) sont testés avec IFN-β2 exprimé par des cellules CHO.

19. Procédé pour l'immunopurification d'IFN-β2 humain biologiquement actif, comprenant les étapes suivantes : faire passer un échantillon contenant IFN-β2 humain à travers une colonne d'immunoadsorption contenant un anticorps monoclonal selon la revendication 1, lié à un support de phase solide, laver la colonne et éluer IFN-β2 de la colonne.

## Revendications (Revendications pour l'(les) Etat(s) contractant(s) suivant(s): ES)

1. Procédé pour produire une lignée de cellules hybridomes capables de produire un anticorps monoclonal, lequel anticorps monoclonal est capable de se lier spécifiquement à l'interféron-β2 (IFN-β2) humain naturel ainsi qu'à IFN-β2 humain recombiné exprimé par des cellules de mammifères et avec IFN-β2 humain recombiné exprimé par des cellules bactériennes, capable d'inhiber l'activité biologique de IFN-β2 humain lorsqu'il y est lié et capable de libérer IFN-β2 qui lui est lié par élution avec de l'acide citrique 50 mM, dans lequel des cellules de myélome sont fusionnées avec des cellules de rate en provenance d'une souris préalablement immunisée avec IFN-β2 humain recombiné exprimé par des cellules bactériennes ou avec une protéine de fusion recombinée contenant IFN-β2 humain exprimé par des cellules bactériennes, et une lignée de cellules hybridomes est sélectionnée pour la production d'un anticorps monoclonal qui se lie à IFN-β2 humain recombiné exprimé par des cellules de mammifères.

2. Procédé selon la revendication 1, dans lequel les cellules de myélome sont des cellules de myélome de souris et les cellules de rate proviennent d'une souris immunisée avec une protéine de fusion recombinée contenant IFN-β2 humain.

3. Procédé selon la revendication 2, dans lequel la protéine de fusion est la Protéine A-IFN-β2.

4. Procédé selon la revendication 1, comprenant les étapes suivantes :
(a) immuniser des souris avec IFN-β2 humain ou avec une protéine de fusion contenant IFN-β2 humain, la source dudit IFN-β2 ou de ladite protéine de fusion étant l'une des sources sélectionnées dans le groupe comprenant une protéine naturelle, une protéine recombinée produite dans des cellules bactériennes et une protéine recombinée produite dans des cellules de mammifères ;
(b) fusionner les cellules de rate en provenance desdites souris avec des cellules de myélome de souris en la présence d'un promoteur de fusion approprié ;
(c) tester le supernageant des cellules fusionnées cultivées en ce qui concerne la présence des anticorps monoclonaux recherchés avec IFN-β2 humain provenant d'une desdites sources différente de celle de l'IFN-β2 utilisé dans l'étape (a) ci-dessus ; et
(d) sélectionner et cloner la lignée de cellules hybridomes produisant les anticorps monoclonaux recherchés.

5. Procédé selon la revendication 4, dans lequel, dans l'étape (a), les souris sont immunisées avec la Protéine A-IFN-β2 exprimée par *E. coli* et les surnageants d'hybridomes de l'étape (c) sont testés avec IFN-β2 exprimé par des cellules CHO.

6. Lignée de cellules hybridomes CNCM I-813 (n° 34-1).

7. Procédé pour obtenir un anticorps monoclonal capable de se lier spécifiquement à l'interféron-β2 (IFN-β2) humain naturel ainsi qu'à IFN-β2 humain recombiné exprimé par des cellules de mammifères et avec IFN-β2 humain recombiné exprimé par des cellules bactériennes, capable d'inhiber l'activité biologique de IFN-β2 humain lorsqu'il y est lié, et capable de libérer IFN-β2 qui lui est lié par élution avec 50 mM d'acide citrique, le procédé comprenant les étapes suivantes :
(a) immuniser des souris avec IFN-β2 humain ou avec une protéine de fusion contenant IFN-β2 humain, la source dudit IFN-β2 ou de ladite protéine de fusion étant l'une des sources sélectionnées dans le groupe comprenant : une protéine naturelie, une protéine recombinée produite dans des cellules bactériennes et une protéine recombinée produite dans des cellules de mammifères ;
(b) fusionner les cellules de rate en provenance desdites souris avec des cellules de myélome de souris en la présence d'un promoteur de fusion approprié ;
(c) tester le surnageant des cellules fusionnées cultivées en ce qui concerne la présence des anticorps monoclonaux recherchés avec un IFN-β2 humain provenant d'une desdites sources différente de celle de l'IFN-β2 utilisé dans l'étape (a) ci-dessus ; et
(d) sélectionner et cloner la lignée de cellules hybridomes produisant les anticorps monoclonaux désirés et soit (i) cultiver la lignée de cellules hybridomes sélectionnée dans un milieu de croissance approprié et récupérer les anticorps monoclonaux recherchés du surnageant, soit (ii) injecter la lignée de cellules hybridomes sélectionnée dans des souris et récupérer les anticorps monoclonaux désirés du fluide ascitique desdites souris.

8. Procédé selon la revendication 7, dans lequel l'anticorps est de la classe IgG1.

9. Procédé selon la revendication 7, dans lequel, dans l'étape (a), les souris sont immunisées avec IFN-β2 humain recombiné exprimé par des cellules bactériennes, ou avec une protéine de fusion recombinée contenant IFN-β2 humain exprimé par des cellules bactériennes, et les surnageants d'hybridomes de l'étape (c) sont testés avec IFN-β2 humain recombiné exprimé par des cellules de mammifères.

10. Procédé selon la revendication 7, dans lequel, dans l'étape (a), les souris sont immunisées avec la Protéine A-IFN-β2 exprimée par *E. coli* et les surnageants d'hybridomes de l'étape (c) sont testés avec IFN-β2 exprimé par des cellules CHO.

11. Procédé pour produire des anticorps monoclonaux anti-IFN-β2, qui comprend les étapes suivantes : cultiver l'hybridome CNCM I-813 dans un milieu de croissance approprié et récupérer les anticorps monoclonaux du surnageant dudit hybridome.

12. Anticorps monoclonal pouvant être exprimé par la lignée de cellules hybridomes CNCM I-813 (HB2 34-1).

13. Procédé pour produire des anticorps monoclonaux anti-IFN-β2, qui comprend les étapes suivantes : injecter l'hybridome CNCM I-813 dans une souris et récupérer les anticorps monoclonaux du fluide ascitique de ladite souris.

14. Procédé pour l'immunopurification d'IFN-β2 humain biologiquement actif comprenant les étapes suivantes : faire passer un échantillon contenant IFN-β2 humain à travers une colonne d'immunoadsorption contenant un anticorps monoclonal produit selon la revendication 7 lié à un support de phase solide, laver la colonne et éluer IFN-β2 de la colonne.
